# EUROPEAN PATENT APPLICATION

(11) **EP 4 837 973 A1**
(43) Date of publication of application: **11.11.2026**
(21) Application number: 25175516.1
(22) Date of filing: 09.05.2025
(51) Int. Cl.: C12Q 1/6883, C12Q 1/6886

(54) **IN VITRO METHOD AND KIT FOR PROVIDING INFORMATION FOR PREDICTING A SUBJECT 'S RESPONSIVENESS TO IMMUNOTHERAPY AND/OR THE SUBJECT 'S LIKELIHOOD OF DEVELOPING OR PROGRESSING WITH A DISEASE**

(71) Applicant: Ludwig-Maximilians-Universität München, 80539 München (DE)
(72) Inventor: HEINZERLING, Lucie, 80337 München (DE)
(74) Representative: Hoefer & Partner Patentanwälte mbB

(57) **Abstract**

The invention concerns an *in vitro* method for providing information for predicting a subject's responsiveness to and/or benefit from immunotherapy and/or the subject's likelihood of progression, occurrence or recurrence of disease, comprising: a) generating a stool-based sample of the subject; b) stimulating cells comprising or consisting of peripheral blood mononuclear cells (PBMCs) with the stool-based sample; c) performing a gene expression analysis of the stimulated PBMCs; and d) using the results of the gene expression analysis as said information.

## Description

### Field of the Invention

The invention concerns an in vitro method for providing information for predicting a subject's responsiveness to or benefit from immunotherapy and/or the subject's likelihood of development, progression, or recurrence or occurrence of disease. The invention furthermore concerns a kit as well as uses of said method.

### Background of the Invention

In the field of immunotherapy, the problem persists in that no known markers or indicators exist which can be used to accurately predict possible responsiveness or benefit of a subject to the immunotherapy.

For example, in cancer immunotherapy, established response markers include tumor mutational burden (TMB), programmed death-ligand 1 (PD-L1) expression, presence of tumor-infiltrating lymphocytes, or mismatch repair deficiency. These, however, lack precision, are only valid in few tumor entities and rely on tumor samples. Currently, they are not used in standard routine care to decide on therapy in e.g., melanoma.

Studies have shown that the gut microbiome of the patient including its diversity and composition determines response to immunotherapy and toxicity (Zitvogel et al. Nature Medicine, 2025, in press). Predicting immunotherapy response by assessing a patient's baseline gut microbiome composition by metagenomics has found limited reproducibility of the results based on microbial signatures. Thus, as of now gut microbiome signatures cannot reliably determine response.

The known attempts for predicting responsiveness are thus inaccurate, irreproducible or unreliable, and unsuitable for clinical practice.

### Summary

It is an object of the present invention to overcome these deficiencies. In particular consideration of these deficiencies, it is an object of the present invention to provide a method for providing information for accurately predicting a subject's responsiveness to or benefit from immunotherapy and/or the subject's likelihood of progression, recurrence or occurrence of disease. Furthermore, it is an object of the present invention to provide a kit which can provide such information. It is another object of the present invention to provide uses of the method and/or stool-based samples from a subject for predicting response to or benefit from immunotherapy of a patient, classifying/predicting the likelihood of a patient responding to immunotherapy, and/or selecting a mode of treatment for the patient, for obtaining such information, and/or for using such information to classify responsiveness, likelihood of progression, recurrence or occurrence of disease.

The solution of these objects is achieved by the subject matter of the independent claims. The dependent claims contain advantageous embodiments of the present invention.

In particular, the solution of at least one of these objects is achieved by the method according to claim 1. The method is *in vitro,* and is suitable for and/or geared towards providing information for predicting a subject's responsiveness to or benefit from immunotherapy and/or the subject's likelihood of progression, recurrence or occurrence of disease. The method comprises the following:
a) generating a stool-based sample of the subject;
b) stimulating cells comprising or consisting of peripheral blood mononuclear cells (PBMCs) with the stool-based sample or parts thereof;
c) performing a gene expression analysis of the stimulated PBMCs;
d) using the results of the gene expression analysis as said information.

Thereby, leveraging gut microbiota present in stool-based samples, such as stool cultures, to gain information for predicting immunotherapy response by determining its potential for activation or deactivation of PBMCs allows better treatment decisions. Employing gene expression analysis on the stimulated PBMCs, as opposed to, for example, metagenomics of the stool sample directly, provides information on effects of the gut microbiome, which affects, especially enhance or suppress, immune cell activation and function, thereby contributing to observed clinical responses. Using PBMCs as effector cells for stimulation integrates many relevant factors and interactions of an immune response highly relevant for effectiveness, benefit and toxicity of potential immunotherapies. Using stool-based samples as a stimulant at the same time integrates many relevant factors and interactions related to the gut microbiome of a subject. Combining these two elements, i.e. the use of PBMCs and a stool-based sample for stimulation thereof allows obtaining information highly relevant for the subject's expected responsiveness to or benefit from immunotherapy and/or the subject's likelihood of progression, recurrence or occurrence of disease.

The method according to claim 1 thus provides suitable, accurate, reproducible, and reliable information which can be used in predicting a subject's responsiveness to or benefit from immunotherapy and/or the subject's likelihood of progression, recurrence or occurrence of disease, in particular an immune-related disease or cancer. It thus covers both treatment and prevention. Further yet, the method is carried out *in vitro,* which strongly simplifies its use in clinical and/or lab-at-home applications.

Benefit in the sense herein preferably means or concerns any form, or indication thereof, that a subject benefits from the treatment, also apart from responsiveness, in particular relating to a prolonged subject's overall survival (OS) or progressive free survival (PFS) of the subject. In particular, there are some diseases, such as cancers, in which a subject does not directly respond to a therapy (such as tumor growth not inhibited), but in which the OS or PFS of the subject is increased/prolonged.

Preferably, the foregoing method steps a - d are carried out in the order of a, b, c, d. In some embodiments, sub-steps are preferably carried out in addition to the aforementioned a - **d,** wherein preferably the overlaying order remains a, b, c, d. Such sub-steps, in addition to further preferable embodiments below, preferably comprise preparation steps and/or transfer steps.

Preferably, the method performs in step c gene expression analysis on the cells comprising or consisting of PBMCs or PBMC subsets stimulated with the stool-based sample. In other words, preferably, the present method step c does not comprise performing gene expression analysis on the stool-based sample directly and/or performing gene expression analysis directly on other samples, such as PBMCs without stool-based sample stimulation, tissue samples, hair samples, tumor samples, fluid samples, breath samples, blood samples, urine samples, etc., taken or retrieved from the subject. In alternative embodiments, one or more of the above can be used in supplementation of the present method as a further step in addition to said steps a - d.

Advantageously, the stool-based sample is a stool sample or a cultured stool sample, or a fraction thereof, in particular a supernatant fraction thereof. Preferably, in the supernatant fraction of the stool-based sample, especially of the cultured stool sample, whole cells are filtered out (i.e. cell-free) during the generation of the stool-based sample of the subject. Preferably, cell-fragments, peptides, metabolites, ligands, and the like, or a combination thereof are included in the supernatant fraction. Methods for obtaining a stool sample, suspending and cultivating it are known in the art and can be used according to the present invention. Preferably, any cultivating of a stool (based) sample is done under anaerobic conditions. Standard tools and kits as commercially available may also be used.

Preferably, whole blood or a fraction thereof is used as the cells comprising or consisting of peripheral blood mononuclear cells (PBMCs). According to a preferred embodiment of the invention, the cells comprising or consisting of PBMCs are comprising a significant fraction of peripheral blood mononuclear cells. According to one embodiment, whole blood samples can be used. Preferably, a fraction of whole blood is used in which the fraction of PMCS is enhanced as compared to whole (peripheral) blood or a subset of PBMC.

PBMCs include lymphocytes (T cells, B cells, NK cells) monocytes and dendritic cells, whereas erythrocytes and platelets have no nuclei, and granulocytes (neutrophils, basophils, and eosinophils) have multi-lobed nuclei. In humans, lymphocytes make up the majority of the PBMC population, followed by monocytes, and only a small percentage of dendritic cells. The isolation of the cells comprising or consisting of PBMCs may be performed by any suitable method known in the art. For example, PBMCs can be extracted from whole blood using Ficoll, a hydrophilic polysaccharide that separates layers of blood, and gradient centrifugation, which will separate the blood into a top layer of plasma, followed by a layer of PBMCs (buffy coat) and a bottom fraction of polymorphonuclear cells (such as neutrophils and eosinophils) and erythrocytes. Preferably, the cells comprising or consisting of PBMCs as used in the present invention comprise at least lymphocytes, in particular lymphocytes (T cells, B cells, NK cells) monocytes and dendritic cells. According to one preferred embodiment, the cells comprising or consisting of PBMCs may be obtained from a healthy donor, in particular a donor not having cancer or an immune-related disease as defined herein. According to one embodiment, the cells comprising or consisting of PBMCs are from the same subject or patient from which the stool-related sample is obtained. According to one preferred example, the cells comprising or consisting of PBMCs comprise or consist of healthy donor PBMCs, with an exemplary concentration of 1×10⁶ cells/ml. Preferably, a healthy donor is defined via common principles in donor selection, and is particularly not a responder or non-responder.

In some examples, a stool sample is obtained from the subject, dispersed in a liquid medium, optionally cultured, and a supernatant thereof is obtained and used in step b) of the foregoing. Therein, the supernatant is preferably cell-free and/or sterile-filtered.

In preferable examples, the stool-based sample, in particular the supernatant, is used in the stimulating according to step b) at a concentration of 0.1 to 20 vol.-%, in particular 1 to 10 vol.- %, based on the total volume of the sample containing the cells comprising or consisting of peripheral blood mononuclear cells (PBMCs).

Preferably, the stimulating of step b) is performed from about 0.1 h to about 48 h, in particular from about 1 h to 24 h. In some examples, stimulation was carried out for about 24 h. A surprising advantage of the relatively short stimulation time needed is that the stool-based sample may even not be sterile (not subjected to sterile filtration) or cell free. Thus, due to the relatively short stimulation time a contamination by cells still present in the stool-based sample used for PBMC stimulation is neglectable and will not significantly influence the results of gene expression analysis of the stimulated PBMCs (using suitable primers/markers).

Preferably, the stimulating of step b) is performed in an incubator at a temperature suitable for cultivation of PBMCs, known in the art, in particular about 37°C, and especially for about 24 h.

In some embodiments, the stimulating of the PBMCs, especially aforementioned step b), is performed in the presence of further stimulans or stimulating agent(s) in addition to the stool-based sample.

The present method is suitable for providing information for predicting a subject's responsiveness to a variety of immunotherapies, i.e. the responsiveness of one certain subject to a variety of immunotherapies or benefit from a variety of immunotherapies and/or responsiveness/benefit of multiple subjects to respectively one or a variety of immunotherapies.

Immunotherapies and known in the art and are for example defined and illustrated in Waldman, A.D., Fritz, J.M. & Lenardo, M.J.: "A guide to cancer immunotherapy: from T cell basic science to clinical practice", Nat Rev Immunol 20, 651-668 (2020). https://doi.org/10.1038/s41577-020-0306-5, or in Kavita A. Iyer et al.: "Emerging Targets and Therapeutics in Immuno-Oncology: Insights from Landscape Analysis", Journal of Medicinal Chemistry 2024 67 (11), 8519-8544, DOI: 10.1021/acs.jmedchem.4c00568; or in Emens LA, Romero PJ, Anderson AC, et al.: "Challenges and opportunities in cancer immunotherapy: a Society for Immunotherapy of Cancer (SITC) strategic vision", Journal for ImmunoTherapy of Cancer 2024;12:e009063. doi:10.1136/jitc-2024-009063.

Preferably, such immunotherapies include immune checkpoint inhibitors (ICI), bispecific antibodies (Bispecifics), T cell receptors against cancer (TAC), cellular therapies such as chimeric antigen receptor cell therapy (CAR-T-cell), and vaccines, as known in the art.

Preferably, one immunotherapy is a cancer immunotherapy, in particular a checkpoint inhibitor therapy.

Preferably, the disease, in particular the immune-related disease is a cancer, an inflammatory disease such as inflammatory bowel disease, a metabolic syndrome, or an autoimmune disorder.

Preferably, the cancer, particularly that of the subject and that of which the responsiveness to or benefit from immunotherapy is to be predicted and/or that of which the likelihood of progression, recurrence or occurrence of disease, concerns a subject's present or possibly developing primary cancer.

Further preferably, the cancer is one or more selected from the group consisting of melanoma, lung cancer, cholangiocarcinoma, colon cancer, adenocarcinoma, rectal cancer, breast cancer, synovial sarcoma, chondrosarcoma, thyroid cancer, stomach cancer, thymus cancer, cervical cancer, glioma, brain cancer, bladder cancer, prostate cancer, leukemia, kidney cancer, liver cancer, colorectal cancer, pancreatic cancer, ovarian cancer, lymphoma, uterine cancer, oral cancer, bronchial cancer, nasopharyngeal cancer, laryngeal cancer, skin cancers, blood cancer, parathyroid cancer, and ureteral cancer.

Preferably, the immunotherapy, the responsiveness of the patient which is to be predicted, is one for the treatment of one or more diseases or disorders selected from the above. Preferably, the immunotherapy, the benefit of the immunotherapy to the patient which is to be predicted, is one for the treatment of one or more diseases or disorders selected from the above.

Methods for performing the gene expression analysis of the stimulated PBMCs are known in the art and also commercially available. Advantageously, the gene expression analysis performed is a gene expression analysis allowing quantification of gene expression, in particular quantification using quantitative real-time PCR (qRT-PCR) or RNA sequencing or any other known method allowing convenient gene expression analysis of the stimulated PBMCs. In preferable examples, RNA gene expression analysis is performed using a counter, for example an nCounter SPRINT Profiler (NanoString Technologies, Inc.), and preferably "nCounter PanCancer Immune Profiling Panel^{™} (human) (Nanostring, XT_PGX_HuV1_CancerImmune_CSO)", with 770 analyzed genes.

Preferably, RNA from the stimulated PBMCs is isolated using an RNA isolation kit, for example "QIAGEN RNeasy Mini Kit (QIAGEN)", especially with added b-Mercaptoethanol, for example about 10 µl. Further, as a preferred example, roughly 75ng of RNA are used for hybridization for 20h, preferably using a thermal cycler. In one example, a "BIORAD, C1000 Touch Thermal Cycler" was used.

Advantageously, the gene expression analysis performed comprises but is not limited to analysis of genes involved in tumor proliferation, immune response, angiogenesis, and apoptosis. Thereby, advantageously, genes contributing to this method are involved in for example a complement pathway, an interferon pathway, immune signaling and immune regulation.

The term "stimulated PBMCs" as used herein and in the claims is used as a synonym for "stimulated cells comprising or consisting of PBMCs or subsets thereof". The term "stool-based sample" as used herein and in the claims is used as a synonym for "stool samples or stool-culture derived samples or parts thereof". After the stimulation with the stool-based sample, the cells comprising or consisting of PBMCs or subsets thereof may be used for gene expression analysis as described herein, or they may be further fractionated or treated, in particular for further enrichment or separation of the PBMCs.

In preferable embodiments, the information used for predicting responsiveness includes the gene expression analysis results for at least one of the group of genes including AIRE (autoimmune regulator), FCGR2A (transporter functions), CCL25 (complement), TAP1 (antigen processing), IFNAR1 (pathogen response), BST2 (cell growth, metastasis) and CSF2RB (cell growth), and/or the gene expression analysis results for at least one of the group of genes including of BCL10 (immune signaling adaptor), CASP3 (cell apoptosis), JAK2 (cytokine and growth factor signaling), IL15 (inflammation) and TNFSF14 (regulation), EGR1 (early growth response), IGF2R (tumor growth, apoptosis, angiogenesis, and invasion) and UBC (cell survival, proliferation, sensitivity to treatment).

Preferably, the foregoing terms in parenthesis, i.e. "(autoimmune regulator)", "(cell growth)", etc. pertain to an effect or involvement of the respective gene in promoting or suppressing responsiveness to the immunotherapy and/or promoting or suppressing progression and/or development or recurrence of disease, for example cancer, and are especially for illustrative and explanatory purposes.

In some preferable examples, the inclusion (e.g. whether or not included) of one or more of these genes, as foregoing, in the gene expression analysis results is preferably determined dependent on said effects and/or their effect strength and/or a different effect thereof. Furthermore, the inclusion of one or more, especially groups, of these genes in the gene expression analysis is preferably determined based on separate, common or interdependent effects of these genes.

Preferably, the method includes the following. First, performing the gene expression analysis as described in above steps a) to c) on one or more patients who are non-responders (henceforth also abbreviated as "NR") to immune therapy in order to obtain one or more NR gene expression signatures. The NR gene expression signature(s) is/are compared to the results of the gene expression analysis of the subject's stool-based sample stimulated PBMCs in accordance with the above. The expected responsiveness of the subject to immunotherapy and/or the likelihood of developing or progressing with an disease, is determined based on a result of said comparison.

In other words, preferably, the method comprises first carrying out steps a, b, c, i.e. generating stool-based sample(s) (a) of a patient known or predetermined or predicted to being NR, stimulating (b) cells comprising or consisting of PBMCs with the stool-based sample of the NR patient, and performing (c) gene expression analysis of the stimulated PBMCs. Then, as described above, the same is carried out with respect to a subject, i.e. generating the subject's stool-based sample (a), stimulating (b) the cells comprising or consisting of PBMCs, and performing (c) gene expression analysis thereon. Then, these results of the gene expression analysis of the NR patient are compared with those of the subject. The expected responsiveness of the subject to immunotherapy and/or the likelihood of developing or progressing with a disease, is then determined based on a result of said comparison. Preferably, in step (d), the information comprises the comparison result.

Advantageously, the method includes the following. First, performing the gene expression analysis as described in above steps a) to c) on one or more patients who are good responders (henceforth abbreviated to "R") to immunotherapy in order to obtain one or more R gene expression signatures. The R gene expression signature(s) is/are compared to the results of the gene expression analysis of the subject's stool-based sample stimulated PBMCs in accordance with the above. The expected responsiveness of the subject to immunotherapy and/or the likelihood of developing or progressing with a disease, is determined based on a result of said comparison.

In other words, preferably and as also with regard to the above concerning NR, the method comprises first carrying out steps a, b, c, i.e. generating stool-based sample(s) (a) of a patient known or predetermined or predicted to being R, stimulating (b) cells comprising or consisting of PBMCs with the stool-based sample of the R patient, and performing (c) gene expression analysis of the stimulated PBMCs. Then, as described above, the same is carried out with respect to a subject, i.e. generating the subject's stool-based sample (a), stimulating (b) the cells comprising or consisting of PBMCs, and performing (c) gene expression analysis thereon. Then, these results of the gene expression analysis of the R patient are compared with those of the subject. The expected responsiveness of the subject to immunotherapy and/or the likelihood of developing or progressing with a disease, is then determined based on a result of said comparison. Preferably, in step (d), the information comprises the comparison result.

Preferably, the foregoing described comparisons of the results of the gene analysis based on an R patient and based on an NR patient are combinable. For instance, the gene analysis result with respect to the subject is compared with the gene expression analysis result of the R patient and the NR patient. Preferably, in step (d), the information comprises both of these respective comparison results. Further preferably, in step (d), it is determined which of the respective comparison results are used as said information, or both are used as said information. For example, one of the comparison results (for example with R patient) can be used to verify the other comparison result (for example with NR patient, and vice versa).

Further preferably, the regard to the above comparison between subject and R, NR, the present method is repeated afterwards for an additional subject. Therein, a recursive or iterative approach is possible, in which the second (or more) subject's corresponding gene expression result is compared with R and NR gene expression signatures, as above, and/or with the result from the first subject. For instance, the first subject is predicted as a responder, and the results from the second subject are compared to those of the first subject for predicting responsiveness and/or development/progression of the second subject. This iterative approach is preferably combinable or employable in a machine learning algorithm, particularly neural network.

In some embodiments, in addition or alternatively to comparisons of a subject's gene expression analyses (resulting from subject's stool stimulated PBMCs) with those of R and/or NR subjects, the R and/or NR subject's gene expression results are preferably collected/pooled, in particular on a storage medium, for training an algorithm, which especially subsequently categorizes or classifies a (new) subject's gene expression result into R and/or NR based on said collected/pooled (prior) R and/or NR subject's gene expression results.

Further advantageously, especially in the above comparison of the subject's gene expression analysis results with those of the R patient and/or of the NR patient, the comparison comprises gene expression analysis results, especially respectively, for at least one of the group of genes including AIRE, FCGR2A, CCL25, TAP1, IFNAR1, BST2 and CSF2RB, and/or for at least one of the group of genes including of BCL10, CASP3, JAK2, IL15 and TNFSF14, EGR1, IGF2R and UBC. Thereby, advantageously, genes associated with effectiveness of certain immunotherapies, particularly used in cancer, are compared between the subject and an R patient cohort and/or an NR patient cohort.

Preferably and especially in an example of comparison with an NR patient as above, the subject is grouped as a predicted responder to immunotherapy and/or having a low risk of progression, recurrence or occurrence of disease if, compared to the NR gene expression signature, the subject's stool-based sample stimulated PBMCs have an upregulated gene expression of at least one of the group of genes consisting of AIRE, FCGR2A, CCL25, TAP1, IFNAR1, BST2 and CSF2RB, and/or the subject's stool-based sample stimulated PBMCs have a downregulated gene expression of at least one of the group of genes consisting of and/or for at least one of the group of genes consisting of BCL10, CASP3, JAK2, IL15 and TNFSF14, EGR1, IGF2R and UBC.

Preferably and especially in an example of comparison of a patient's results with the NR patient cohort as above, the subject is grouped as a predicted non-responder to immunotherapy and/or having a low risk of progression, recurrence or occurrence of disease if, compared to the NR gene expression signature, the subject's stool-based sample stimulated PBMCs have a roughly similar gene expression of at least one of the group of genes consisting of AIRE, FCGR2A, CCL25, TAP1, IFNAR1, BST2 and CSF2RB, and/or the subject's stool-based sample stimulated PBMCs have a roughly similar gene expression of at least one of the group of genes consisting of and/or for at least one of the group of genes consisting of BCL10, CASP3, JAK2, IL15 and TNFSF14, EGR1, IGF2R and UBC.

Further preferably and especially in an example of comparison with an R patient as above, the subject is grouped as a predicted non-responder to immunotherapy and/or having a high risk of developing or progressing with a disease if, compared to the R gene expression signature, the subject's stool-based sample stimulated PBMCs have a downregulated gene expression of at least one of the group consisting of AIRE, FCGR2A, CCL25, TAP1, IFNAR1, BST2 and CSF2RB, and/or the subject's stool-based sample stimulated PBMCs have an upregulated gene expression of at least one of the group consisting of and/or for at least one of the group consisting of BCL10, CASP3, JAK2, IL15 and TNFSF14, EGR1, IGF2R and UBC.

Preferably and especially in an example of comparison of a patient's results with a patient with the R patient cohort as above, the subject is grouped as a predicted responder to immunotherapy and/or having a low risk of developing or progressing with a disease if, compared to the R gene expression signature, the subject's stool-based sample stimulated PBMCs have a roughly similar gene expression of at least one of the group consisting of AIRE, FCGR2A, CCL25, TAP1, IFNAR1, BST2 and CSF2RB, and/or the subject's stool-based sample stimulated PBMCs have roughly similar gene expression of at least one of the group consisting of and/or for at least one of the group consisting of BCL10, CASP3, JAK2, IL15 and TNFSF14, EGR1, IGF2R and UBC.

Preferably, in some examples of comparison between subject, R and/or NR, PBMCs from the same healthy donor are used in the respective stimulating step. In other words, the PBMCs of one healthy donor are separately stimulated with the R stool-based sample and/or the NR stool-based sample, and the subject's stool-based sample, and gene expression analysis is performed on each of these. This has the additional surprising advantage in that donor-specific effects can be excluded. It is to be understood, however, that the foregoing methods are not limited thereto, the PBMCs used for each stimulation may be from different healthy donors, as donor-specific effects are non-significant.

In the above examples for gene expression analysis, at least one of the group consisting of AIRE, FCGR2A, CCL25, TAP1, IFNAR1, BST2 and CSF2RB and/or at least one of the group consisting of BCL10, CASP3, JAK2, IL15 and TNFSF14, EGR1, IGF2R and UBC are included.

Preferably, more than one, for example two or more, three or more, four or more, five or more, six or seven of the genes of the group consisting of AIRE, FCGR2A, CCL25, TAP1, IFNAR1, BST2 and CSF2RB are included. In addition, or alternatively thereto, preferably, more than one, for example two or more, three or more, four or more, five or more, six or more, seven, or eight of the genes of the group consisting of BCL10, CASP3, JAK2, IL15 and TNFSF14, EGR1, IGF2R and UBC are included. For example, in the case of above "additionally", one or more of each group may be included, two or more of each group may be included, or three or more of each group may be included, or more of each group may be included, particularly also all of each group may be included. Further yet, the number of genes from each group is optionally not the same, i.e. two of one group can be included while only one or three or more of another group are included.

According to particular preferred embodiments of the different aspects of the present invention as described herein,
- all of the group consisting of AIRE, FCGR2A, CCL25, TAP1, IFNAR1, BST2 and CSF2RB and/or all of the group consisting of BCL10, CASP3, JAK2, IL15 and TNFSF14, EGR1, IGF2R and UBC are included/comprised in the (results of the) gene expression analysis; or
- all but one of the group consisting of AIRE, FCGR2A, CCL25, TAP1, IFNAR1, BST2 and CSF2RB and/or all but one of the group consisting of BCL10, CASP3, JAK2, IL15 and TNFSF14, EGR1, IGF2R and UBC are included/comprised in the (results of the) gene expression analysis; or
- all but two of the group consisting of AIRE, FCGR2A, CCL25, TAP1, IFNAR1, BST2 and CSF2RB and/or all but two of the group consisting of BCL10, CASP3, JAK2, IL15 and TNFSF14, EGR1, IGF2R and UBC are included/comprised in the (results of the) gene expression analysis.

Preferably, more than one, for example two or more, three or more, four or more, five or more, six or seven of the genes of the group consisting of AIRE, FCGR2A, CCL25, TAP1, IFNAR1, BST2 and CSF2RB are included. In addition, or alternatively thereto, preferably, more than one, for example two or more, three or more, four or more, five or more, six or more, seven, or eight of the genes of the group consisting of BCL10, CASP3, JAK2, IL15 and TNFSF14, EGR1, IGF2R and UBC are included.

It should be noted that the grouping according hereto is preferably chosen based on observed up- and down-regulation of gene expression when comparing R patients with NR patients, i.e. the one group was observed to be down-regulated (BCL10, CASP3, JAK2, IL15 and TNFSF14, EGR1, IGF2R and UBC) and the other group was observed to be up-regulated (AIRE, FCGR2A, CCL25, TAP1, IFNAR1, BST2 and CSF2RB). The grouping however is not limited thereto.

Furthermore, the genes included in the gene expression analysis and/or in the gene expression signature(s) are preferably not limited to the foregoing examples, and additional or alternative genes, especially genes observed as up- and/or down-regulated between R and NR, or between a subject and R/NR, are preferably included in the gene expression analysis and/or the gene expression signature(s).

Preferably, the analysis result of gene expression, particularly with regard to the subject and/or the R patient and/or the NR patient, is a gene expression signature, also referred to as "subject gene expression signature", "R gene expression signature", and "NR gene expression signature". Preferably, each gene expression signature comprises information on the one or more genes included in the gene expression analysis as well as the corresponding count for that gene, and preferably additionally includes an identifier for the respective signature (for example "subject", "R", "NR").

Further preferably and especially before a comparison according to any one of the above examples, the count(s) (i.e. transcript counts) of the gene(s) are normalized in the or each gene expression signature. Preferably, before comparison, the gene expression signatures to be compared are normalized. For example, results are normalized to housekeeping probes. For example, housekeeping probes for normalization were selected by geNorm algorithm as in the NormqPCR R library. Further, p-values of each gene are preferably adjusted by by Benjamini-Hochberg method for false discovery rate estimation.

Preferably, in at least one of the foregoing described comparisons, the respective gene expression signatures, especially normalized and p-value-adjusted (p≤0.05), are compared. In some examples, a fold change indicating a multiplicative factor (for example, two-fold, threefold, etc.) is calculated. Therein, a positive fold change corresponds to increase of expression, while a negative fold change corresponds to a decrease in expression.

In particular, preferably a log2 fold change is calculated. Preferably, the at least one of the foregoing described comparisons is carried out using differential gene expression, known in the art.

Further preferably, in at least one of the foregoing described comparisons, a log2 fold change of 1, i.e. a 2-fold change, is used as a threshold for up-regulated expression, and preferably a log2 fold change of -1, i.e. half-fold change, is used as a threshold for down-regulated expression. In other examples, at least a log2 fold change of 1.5 and/or -1.5 is used as respective thresholds. Further, for example, below these threshold examples and above or equal to log2 fold change of 0 (zero, i.e. no change), the genes are considered to be expressed roughly similar. Thus, according to a preferred embodiment of the invention "roughly similar" is used herein in the sense of genes expressed below the above threshold examples and above or equal to log2 fold change of 0 (zero, i.e. no change).

The present invention also concerns a method for classifying the likelihood of responsiveness of a subject to immunotherapy and/or the likelihood of progression, recurrence or occurrence of disease. The method comprises the following. Performing (e) the method according to any one of the foregoing explanations in order to obtain information for predicting responsiveness and/or the likelihood of developing or progressing with a disease. Using the information (f) for predicting responsiveness and/or likelihood in order to obtain a response score. Therein, a higher response score is associated with an increased responsiveness to immunotherapy and/or decreased likelihood of developing or progressing with a disease, and a lower response score is associated with a decreased responsiveness to immunotherapy and/or increased likelihood of developing or progressing with a disease. Thereby, a response score indicative of likelihood of responsiveness or likelihood of developing/progressing with a disease is advantageously obtained, which allows easy and quick comparison with other subject's response scores, or with known R and NR patient's response scores.

Preferably, the response score is a numerical value. For instance, the response score is a value between 0 and 100, preferably a positive integer, whole number, or a value between 0 and 1, as a real number.

In some embodiments, the response score is calculated as follows. The gene expression result, i.e. the gene expression signature, is attained, indicating gene and count. Preferably, the gene expression signature is normalized, as described for example above. Then, the counts of the gene expression signature are added, preferably respectively together with weighted values assigned thereto. For example, certain genes, particularly of the groups discussed above, can be assigned a higher weight-value than others (for example, if these are known to being more relevant in predicting responsiveness and/or development/progression). The weighted values are then summed, and a result response score is attained.

In some examples, the response score is preferably calculated using a machine learning algorithm, for example a neural network. Therein, preferably, a neural network for calculating a response score is trained with input data, preferably gene expression counts (raw and/or normalized and/or p-adjusted/corrected expression levels of genes across samples). Preferably, labels, preferably target outputs (such as condition labels or specific scores correlating with R, NR), are used in supervised learning. The model contains weights, preferably learnable parameters that adjust the strength of connections between neurons, and biases, preferably learnable parameters that allow flexible adjustments to neuron outputs. Activation functions, preferably non-linear functions like ReLU or sigmoid, introduce nonlinearity into the network. A loss function, preferably mean squared error or cross-entropy, quantifies the difference between predicted and actual outputs. An optimizer, preferably stochastic gradient descent, adjusts weights and biases to minimize the loss. Finally, a learning rate, preferably a hyperparameter that controls the magnitude of weight updates, guides the training process. These elements are iteratively adjusted to learn the best model for predicting/estimating response scores.

The trained neural network is then preferably used to calculate a response score based on a subject's gene expression signature.

For example, gene expression signatures for each subject, R, NR patient, are represented as a vector of expression levels. Each element in this vector corresponds to the normalized expression count of a specific gene in that sample (e.g., RNA-seq data). For example, the label could be a responsiveness score like 2 (NR), 5 (responder, but low), or 8 (high responder), or a continuous score representing for example disease progression over time.

Preferably, the calculation of response scores according to any one of the following is preferably repeated with respect to the subject and with respect to an R patient and/or an NR patient, with preferably corresponding normalization and weighting. The resulting response scores can then preferably be numerically compared. Preferably, if a calculated difference between two response scores is above a predetermined threshold, the response score is determined to being indicative of R or NR.

Preferably, the comparison can additionally or alternatively be based on a fraction, i.e. a percentage. For example, if the response score of the subject is 60% of the R patient score, it is preferably determined that the subject has a likelihood of 60% of being a responder. Further, for example, multiple thresholds are definable. For instance, if the response score of the subject is 90% of the R patient score, it is preferably determined that the subject is likely a elite responder.

The present invention also concerns a method for selecting the mode of treatment for a subject or patient. The method comprises using the response score generated in the above as follows: providing an immunotherapy to the subject or patient if the subject or patent has a "high" response score; or providing an alternative therapy other than immunotherapy or a therapy additional to immunotherapy to the subject or patient if the subject or patient has a "low" response score.

The term "providing" as used above encompasses "selecting for". Thus, according to one embodiment, the present invention further concerns a method for selecting the mode of treatment for a subject or patient, wherein the method comprises using the response score generated in the above as follows: selecting the subject or patient for future administration of an immunotherapy to the subject or patient if the subject or patent has a "high" response score; or selecting the subject or patient for future administration of an alternative therapy other than immunotherapy or a therapy additional to immunotherapy to the subject or patient if the subject or patient has a "low" response score.

Preferably, the terms "high" and "low" response score concern a predetermined threshold. The predetermined threshold is preferably based on values obtained by determining the response score of R patients and/or NR patients. These response scores are then preferably ranked, for instance from highest to least. The predetermined threshold is preferably set based on corresponding response scores of the R patients and NR patients. For example, the threshold is preferably set as "high" for a response score higher than the highest response score of NR patients, and higher than or equal to the lowest response score of R patients, and vice versa for "low" response scores.

The present invention also concerns a kit for *in vitro* determination of a gene expression profile of cells comprising or consisting of peripheral blood mononuclear cells (PBMCs) stimulated with a stool-based sample, the kit comprising: reagents for obtaining a stool-based sample from a subject; reagents for culturing cells comprising or consisting of peripheral blood mononuclear cells (PBMCs) stimulated with the stool-based sample; reagents for determining gene expression profiles of the PBMCs stimulated with the stool-based sample; instructions for generating information for predicting a subject's responsiveness to immunotherapy and/or the subject's likelihood of developing or progressing with a disease.

Preferably, the kit comprises instructions for generating a response score for immunotherapy from the gene expression profiles of the PBMCs stimulated with the stool-based sample.

The invention further concerns a use of the method according to one of the foregoing examples and/or a kit according to one of the foregoing examples for predicting the response of a patient to immunotherapy and/or classifying the likelihood of a patient responding to immunotherapy and/or selecting the mode of treatment for the patient.

The invention further concerns a use of a stool-based sample from a subject for stimulating cells comprising or consisting of peripheral blood mononuclear cells (PBMCs) and obtaining a gene expression analysis of the stimulated PBMCs.

Preferably, in the aforementioned use, the results of the gene expression analysis are used as information within the prediction of and/or classification of the responsiveness of the subject for immunotherapy and/or the likelihood of developing or progressing with a disease and/or for selecting the mode of treatment of the subject.

Preferably, in the foregoing, responders R and non-responders NR are pre-defined. For example, in cancer patients and cancer immunotherapy, the Response Evaluation Criteria in Solid Tumors (RECIST) is used for classification into R and NR.

For example, responder patients (R) were pre-defined as those with favorable outcomes, characterized preferably by at least one or more of: an overall survival (OS) of over 40 months, progression-free survival (PFS) exceeding 40 months, a best response of complete response (CR) or stable disease (SD), and no primary resistance to therapy. Primary resistance is preferably defined as early disease progression or lack of response to treatment. On the other hand, non-responder patients (NR) were pre-determined as those who exhibited poor treatment outcomes, including preferably at least one or more of: OS shorter than 40 months, PFS less than 40 months, a best response of progressive disease (PD), or primary resistance to therapy.

Preferably, the foregoing criteria are independent from type of therapy and especially independent from underlying disease, especially cancer type. For example, R and NR are preferably categorized based on one or more of the following: thresholds of response (CR=complete response or PR=partial response), non-response (PD= progressive disease) or SD=stable disease, overall survival (OS), and progression free survival (PFS). With respect to different underlying diseases and/or immunotherapies, the thresholds are preferably pre-determined based on corresponding expectations and experiences.

Preferably, a NR is defined as not meeting any criteria, or not meeting at least one or at least two or at least three criteria for R, and vice versa.

Preferably, the method of pre-determining, setting and/or measuring degree of criteria met, as well as classifying healthy, R and NR patients is carried out before, and especially separate from the foregoing described method for providing information for predicting a subject's responsiveness to immunotherapy and/or the subject's likelihood of developing or progressing with a disease. In particular, it is to be understood that the method comparing the results of the gene expression analysis of the subject according to the foregoing compares the results of the gene expression analysis of the stimulated PBMCs of that subject with those of pre-determined R and NR patients, thus enabling a determination of responsiveness/progression/development. This is thus preferably independent from the general pre-classification into R and NR, i.e. with which criteria this was pre-determined. In other words, the comparison is preferably to be understood as resulting in information indicative of whether the subject will or would meet the criteria used for pre-classification of R, NR, irrespective of the actual criteria.

In some preferable aspects, the term "elite" patient is synonymously used as "responder" or "R", and "hpp" (High-Potential Progression) patient is used synonymously as "non-responder" or "NR".

The foregoing described preferable embodiments and configurations may be combined. In particular, the methods and their steps may preferably be carried out simultaneously or one-after-the-other.

Further details, advantages, and features of the preferred embodiments of the present invention are described in detail with reference to the figures. Therein:

### Brief Description of the Drawings

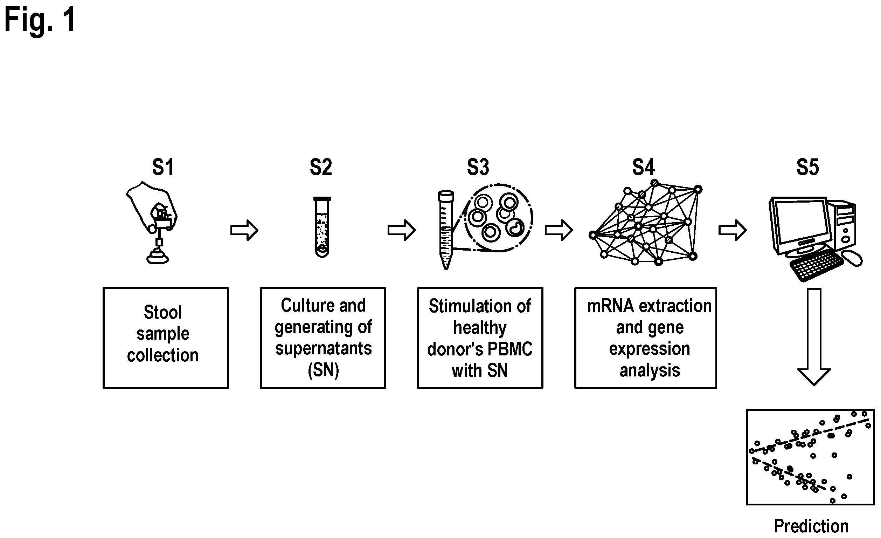

Fig. 1 shows a schematic overview of the steps of obtaining results from gene expression analysis of stimulated PBMCs, i.e. PMBCs stimulated using a stool-based sample (here: cultivated stool sample) according to the present invention from a subject. Herein, steps S1 and S2 are obtaining a stool-sample from the subject, and generating a stool-based sample of the subject, especially a supernatant, step S3 is stimulating cells comprising or consisting of peripheral blood mononuclear cells (PBMCs) with the stool-based sample of S2, step S4 is performing a gene expression analysis of the stimulated PBMCs, step S5 is using the results of the gene expression analysis as information for predicting the subject's responsiveness to immunotherapy and/or the subject's likelihood of developing or progressing with a disease.

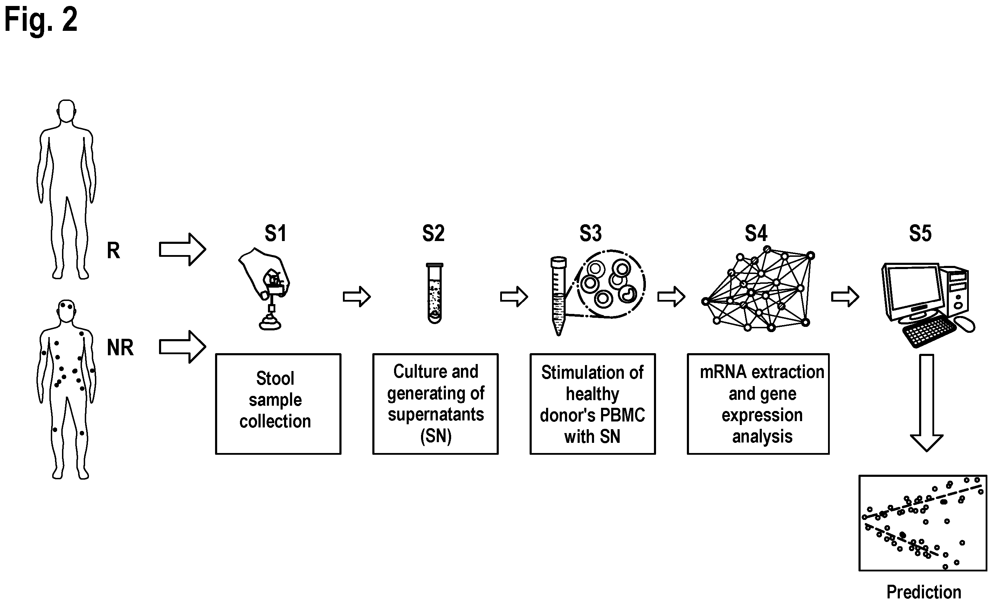

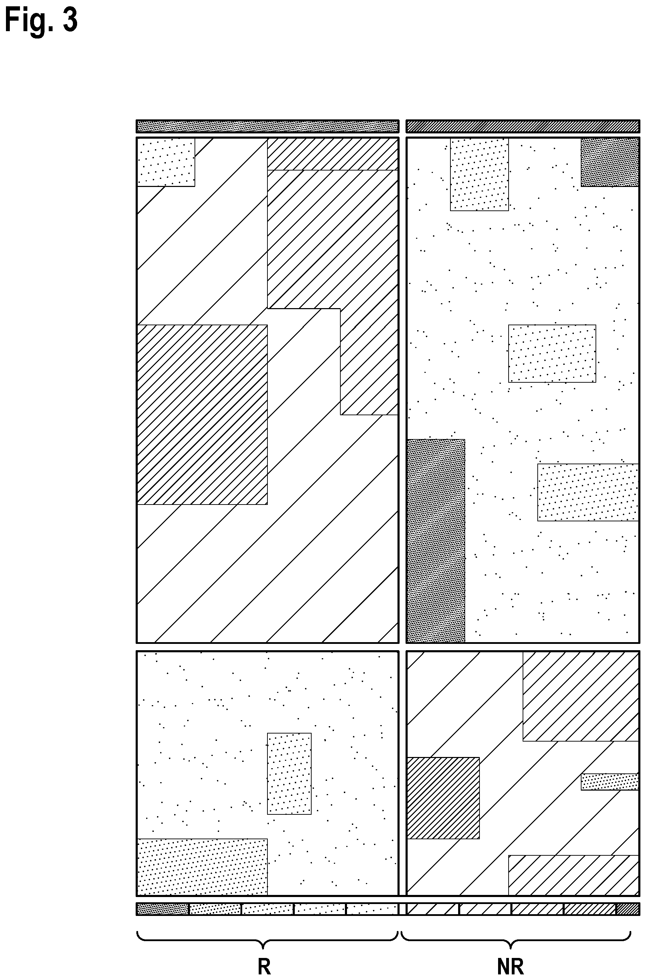

Fig. 2 shows a schematic overview of the steps of obtaining results from gene expression analysis of stimulated PBMCs, i.e. PMBCs stimulated using a stool-based sample (here: cultivated stool sample) according to the present invention from a Responder (R) or non-responder (NR) patient. As described herein, instead of R or NR patients, any subject can be used in order to provide information for predicting the subject's or patient's responsiveness to immunotherapy and/or the subject's likelihood of developing or progressing with a disease. The analysis and comparison of R and NR patients showed a remarkable and surprising difference and distinction in a heat map on the results of the gene expression analysis, as shown in Fig. 3 below.

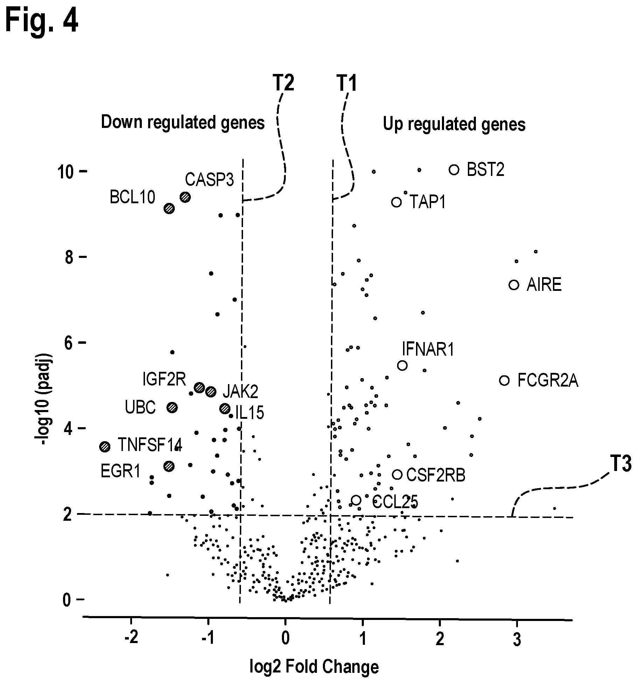

Fig. 3 shows the differential gene expression (DGE) inducted in PBMCs stimulated with a stool-based sample from NR and R patients according to Fig. 2. The results are visualized in the form of a heat map. The clear distinction between the NR and R patterns can be seen. Therein, the heat map is split into top and bottom, wherein the top shows up-regulated genes and the bottom shows down-regulated genes. Further, the heat map is split into left and right, wherein the left shows R patients, or elite patients, and the right shows NR patients, or hpp (High-Potential Progression) patients. Dotted fields are associated with R patients, and line-hashed fields are associated with NR patients. As can be seen therein, when compared to one another, there are clear and distinctive genes being up-regulated and down-regulated with regard to R or NR patients. This is also further shown in Fig. 4.
Fig. 4 shows the change of gene expression in stimulated PBMCs of a R patient according to Figs. 2 and 3 compared to a NR patient according to Figs. 2 and 3 in the form of a volcano plot. The upregulated and downreguated genes are shown and indicated as described herein. The graph plots log2 fold change in gene expression versus -log10(padj), i.e. the adjusted p-value. A first threshold T1 denotes a threshold for determination of upregulation, a second threshold T2 denotes a threshold for determination of downregulation, and a third threshold denotes a threshold of adjusted p-value, particularly a threshold for significance.
   Therein, significantly up- and downregulated genes after SN-*elite* or R patient stimulation of healthy PBMCs, in comparison SN-*hpp* or NR patients in a Volcano plot. Top 10 significantly differentially expressed genes displayed, adjusted p-value (p-adj) used for classification. Empty circles: upregulated after SN stimulation, Filled circles: downregulated after SN.

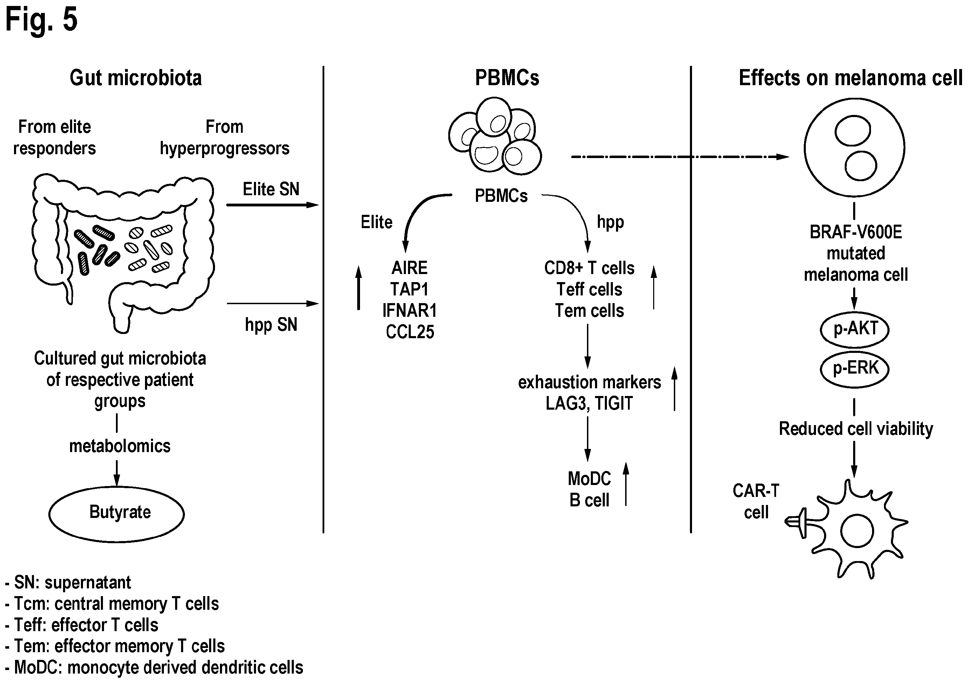

Fig. 5 shows a flow diagram explaining a method according to an embodiment of the present invention, in which exemplary supernatant from elite patients and from hpp (High-Potential Progression) patients are used for stimulating PBMCs. In the middle of Fig. 5, up- and down-regulated genes are compared between elite and hpp patient's supernatant-stimulated PBMCs. In the top or right column of Fig. 5, exemplary explanations of the results are discussed, allowing use thereof for prediction.

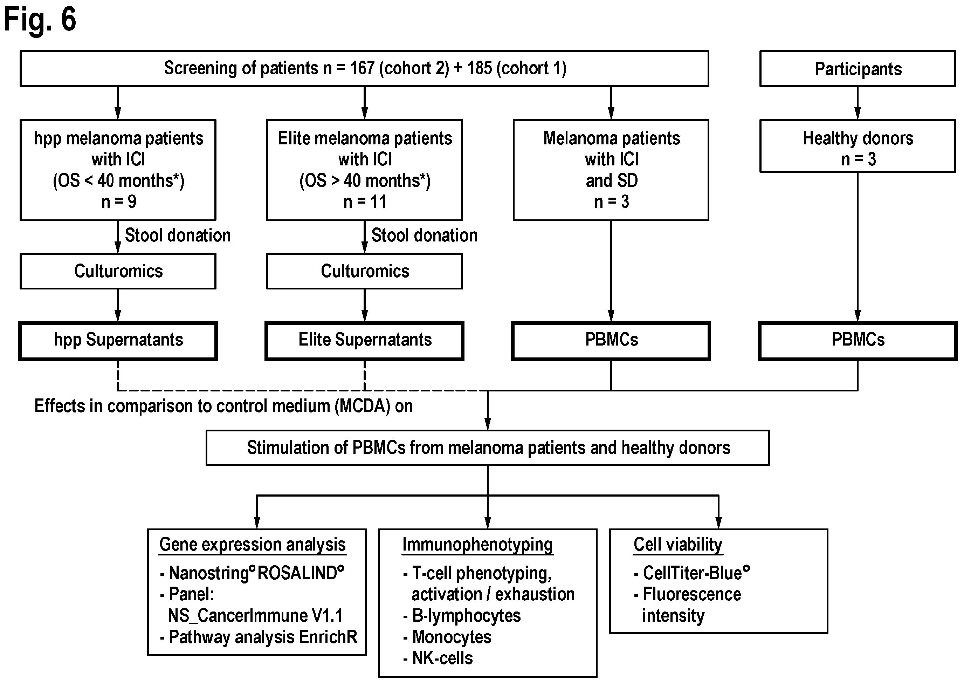

Fig. 6 shows a flow diagram explaining analyses of effects of elite supernatants. Screening of melanoma patients with immune checkpoint-inhibitor treatment (ICI) in the University hospitals Erlangen (UKER) and LMU Munich (LMU), Germany. Culturomics in Stockholm, Karolinska Insitute, Sweden. Patients with stable disease (SD) according to response evaluation criteria in solid tumors (RECIST 1.1) and immune-related adverse event (irAE). *Overall survival (OS) of more than 40 months after metastatic melanoma stage IV (AJCC 2017). Patients with progressive disease (PD) according to response evaluation criteria in solid tumors (RECIST 1.1) and immune-related adverse event (irAE). *Overall survival (OS) of less than 40 months after metastatic melanoma stage IV (AJCC 2017). UKER = university hospital Erlangen-Nürnberg, LMU = Ludwig-Maximilians university Munich, ICI = immune checkpoint-inhibitor, PBMCs = peripheral blood mononuclear cells.

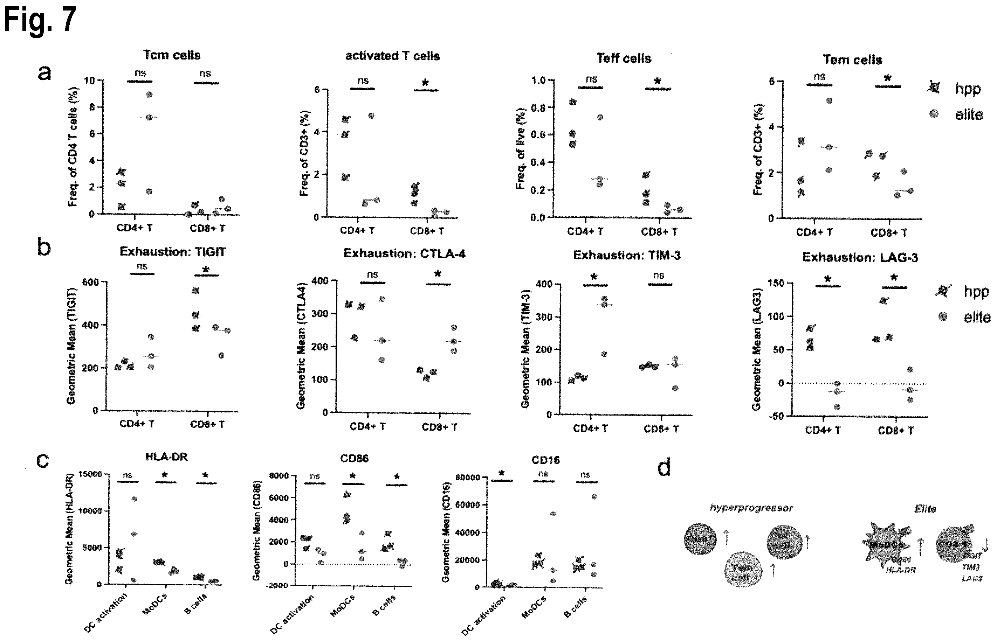

Fig. 7 shows graphs and a diagram for explaining and showcasing results of Immune cell activation and exhaustion markers of peripheral blood mononuclear cells (PBMCs) of healthy donors induced by fecal supernatants from *elite* and *hpp* melanoma patients. Stimulation for 24 h with SN-hpp (SN, purple) and SN-elite (SN, blue). * statistically significant (sign.) in mel., h., or both (p≤0.05). Frequency and/or mean fluorescence intensity (MFI). (a) T-cell phenotype: frequencies, 1st row (left to right): CD4+ regulatory T-cells (T reg) (sign.: h.), CD4+ effector T-cells (sign.: mel., h. almost sign.) and in relation to all lymphocytes (sign.: both). 2nd row: effector CD8+ T-cells in relation to live (sign.: h.) and lymphocytes (%) (sign.: h.), frequency of CD8+ of central memory CD8+ T-cells. (b) T-cell activation/exhaustion markers: 1st row: frequency of CD25+ CD4+ T-cells (sign.: mel.), MFI of CD44 of pan T cells (sign.: mel.), of CD4+ T-cells (sign.: h.). 2nd row: CD44 MFI of CD8+ T-cells (sign.: mel.), frequency of ICOS on pan T-cells (sign.: h.), on CD4+ (sign.: h.) and CD8+ T-cells (sign.: h.). (c) Cellular markers for B-lymphocytes, dendritic cells (DC) activation and monocytic DC (moDC): 1st row: frequency (sign.: both) and MFI (sign.: both) of CD80 on B-cells, MFI of HLA-DR on B-cells (sign.: mel.), frequency of PD-L1 on B-cells (sign.: mel.). 2nd row: frequency of CD86 on B-cells (mel. almost sign.), MFI of PD-L1 on B-cells (sign.: mel., h. almost sign.), and frequency of CD56low/CD16+ in NK cells (sign.: h.). (d) raphic Immune Profils: Summary of immune phenotypes induced by hpp supernatants (SN). hpp SN strongly activate CD8+ T cells, leading to increased proportions of effector (Teff), central memory (Tcm), and effector memory (Tem) T cells. Additionally, hpp SN upregulate exhaustion markers (LAG3, TIGIT, CTLA4) on CD8+ T cells, indicating heightened activation and functional modulation. Enhanced activation of antigen-presenting cells, including monocyte-derived dendritic cells (MoDCs; CD86, HLA-DR) and B cells, highlights the broad stimulatory impact of hpp SN on the immune system. These findings suggest that hpp SN induce a distinct immunophenotype characterized by robust T cell activation, memory formation, and APC activation.
   Therein, the elite and hpp FACS result (healthy patients PBMC) (3 vs 3) shows: Compared to elite SN, hpp SN induce stronger activation of CD8+ T cells, with increased effector and memory T cells HPP supernatants also elevate exhaustion markers (LAG3, TIGIT, CTLA4) and enhance DC and B cell activation (CD86, HLA-DR).
   Abbreviations are: Tcm: central memory T cells; Teff: effector T cells; Tem: effector memory T cells; MoDC: monocyte derived dendritic cells.
Figs. 8 - 11 are supplemental graphs and diagrams showing further results. They will in the following also be referred to as "Supplemental figure" or "suppl. Fig. X", X being 8, 9, 10, or 11.
   In Fig. 8, Elite and hpp FACS result (healthy patients PBMC) (3 vs 3) is shown with: No difference of activated Treg cells, naïve T cell, stem T cell between hpp and elite group, no difference of activation marker (CD27, CD44, ICOS, CD107a, CD28) between hpp and elite group, and no difference of DC and B cell activation (CD80, PD-L1). between hpp and elite group.
   In Fig. 9, Elite and hpp FACS result (melanoma patients PBMC) (3 vs 3) is shown with: Compared to elite SN, hpp SN induce stronger activation of CD8+ T cells, with increased effector and memory T cells, activated Treg cells, decreased central memory T cell.
   In Fig. 10, Elite and hpp FACS result (melanoma patients PBMC) (3 vs 3) is shown with: HPP supernatants also elevate activation markers (CD27, ICOS, CD44, CD107a, CD28) and exhaustion (LAG3, TIGIT).
   In Fig. 11, Elite and hpp FACS result (melanoma patients PBMC) (3 vs 3) is shown with: HPP supernatants also enhance DC and B cell activation (CD86, HLA-DR).

### Detailed Description of the Embodiments

In the following, embodiments of the present invention and in particular a study showcasing proof-of-concept will be described.

### Methods

### Study design

In this prospective proof-of-concept study, effects of gut microbiome-derived supernatants (SN) on PBMCs and melanoma cells were investigated (Fig. 1, Fig. 2, Fig. 5). The institutional review boards of the involved institutions gave ethical approval (LMU Munich No. 20-1122 and 20-1148; University hospital Erlangen No. 120_19 B; EU ethics) and the study followed the standards for Good Clinical Practice and the declaration of Helsinki. All patients gave their written informed consent.

After screening 167 melanoma patients, patients were classified into two groups based on their response to immune checkpoint inhibitor (ICI) therapy at Stage IV. Elite patients were defined as those with favorable outcomes, characterized by an overall survival (OS) of over 40 months, progression-free survival (PFS) exceeding 40 months, a best response of complete response (CR) or stable disease (SD), and no primary resistance to ICI therapy. Primary resistance was defined as early disease progression or lack of response to treatment. On the other hand, HPP (High-Potential Progression) patients were those who exhibited poor treatment outcomes, including OS shorter than 40 months, PFS less than 40 months, a best response of progressive disease (PD), or primary resistance to ICI. Newly diagnosed patients with Stage IV disease showing early progression were also categorized as HPP.

Among the 12 Elite patients, several exhibited exceptional responses to ICI treatment, including CR and SD, with long-term survival outcomes. For example, patient E1, a 38-year-old male with cutaneous melanoma (CM), showed a CR after treatment with lpi or lpi+PD1 or PD1, with a PFS of greater than 134 months and an OS of 134 months. Similarly, other patients, such as E2, E3, and E4, showed long-term survival with favorable responses to ICI therapies like lpi/Pembro or Ipi/Nivo. However, there were some exceptions in the Elite group, such as patient E6, a 37-year-old female with CM, who developed primary resistance to ICI, manifesting as progressive disease (PD) within 0 months of treatment, and her OS was over 109 months. Additionally, patient E7, an 81-year-old male with CM, was monitored in Stage III with progression-free survival, which did not fit the Stage IV analysis but was noteworthy for his stable condition.

On the other hand, the HPP group comprised patients who exhibited poor responses to ICI therapy. For instance, patient H1, a 65-year-old male with CM, showed progression-free survival of only 7 months before developing progressive disease with short overall survival of 30 months, complicated by glioblastoma. Patient H2, a 71-year-old male with CM, showed stable disease (SD) after multiple courses of Pembro, but his survival was notably shorter, and his overall survival was limited to 2 months. Similarly, patient H6, a 76-year-old female with CM, developed PD after just two months of treatment with Pembro, with primary resistance confirmed. Several other HPP patients, including H8 and H9, experienced primary resistance, manifesting as PD and very short survival durations of only 3 months before death.

Special mention is also required for patient H5, an 86-year-old female with CM. This patient was treated with Ipi/Nivo, where she initially responded with partial remission (PR) but later experienced progression after four months, suggesting a delayed response followed by clinical progression. Additionally, the treatment documentation for this patient was incomplete due to therapy starting outside of our institution, but known adverse events such as hepatitis, arthritis, and hypophysitis emerged after two cycles, indicating significant side effects prior to progression. , donated fresh stool for culturomics to produce stable fecal cultures (Table 1). Filtered and cell-free SNs of these cultures were employed to unravel effects on immune and melanoma cells.

Established melanoma cell cultures (MC) of four patients, two with BRAF-V600E mutation and two with wildtype, were used for investigation of SN effects on cell viability, establishing the optimal concentration of SNs in comparison to the control medium (MCDA) as well as the optimal duration of stimulation. By Western Blot, the influences of SNs on protein expression were assessed.

PBMCs were obtained from three healthy volunteers and three melanoma patients (Table 1). Effects of elite and hpp SN on melanoma patients' PBMCs and healthy control's PBMCs regarding immunophenotyping (Fluorescence Activated Cell Sorting, FACS) and gene expression (Nanostring) were compared to control medium (MCDA).

### Generation of supernatants

After receiving feces samples from 12 elite patients and 9 hpp patietns, stools were cultured. Complex gut microbiome culture supernatants for each donated stool were created using 1 to 5g of stool suspended in 10mL PBS with 0.1% L-cysteine and homogenized using a vortexer. Homogenized stool samples were then inoculated in a starter culture using a modified MCDA broth without azeotram and vancomycin [26]. This growing culture is sub-cultured in 1:10 MCDA broth every five to seven days. Stability of cultures has been observed at every subculture time-point with determined stability for one to six weeks (data not shown). Cultures at week two were collected and filtered using a 0.45µM filter to produce the cell-free SNs. All preparations and cultures were done in complete oxygen-free conditions using an anaerobic chamber (85% N2, 10% CO2, 5% H2). Established cell-free SNs of 5 elite patients were used in the following experiments.

### Patient and participant selection

The 12 elite patients and 9 hpp patients donated fresh stool samples and answered a questionnaire including smoking status, treatment with antibiotics or proton-pump inhibitors, intestinal surgeries, probiotics usage, diet (vegetarian, vegan or omnivore) and recent medication including the past four to six weeks. we selected SN-E1 and SN- H1 based on their representative treatment outcomes and their effects on cell viability. Patient E1, an Elite patient, showed a CR and an OS of 134 months, making him an ideal candidate for studying immune mechanisms associated with successful ICI therapy. In contrast, Patient H1 exhibited PD and poor outcomes, reflecting the HPP profile with primary resistance to ICI. These samples represent the extremes of treatment response and are key for investigating immune-related factors influencing treatment success or failure. Additionally, three melanoma patients with stable disease (SD) or partial response (PR) according to the response evaluation criteria in solid tumors (RECIST 1.1) undergoing ICI-therapy and three healthy controls, without diagnosed autoimmune disease or cancer, donated PBMCs. The cutaneous melanoma PBMC donors were two female and one male patient aged 72, 65 and 69 years, respectively. Their stages at ICI-initiation were IIIB for two and once stage IV (AJCC 2017). Two received pembrolizumab and one received ipilimumab and nivolumab combination therapy. The male patient had received prior systemic therapy with BRAF/MEK inhibitors. All three melanoma patients who donated PBMCs developed immune-related adverse events (irAEs) under ICI-therapy. The first was BRAF wildtype, whereas the other two were BRAF-V600E mutated (Table 1).

The healthy PBMC donors comprised two female and one male volunteers without any diagnosed cancer or autoimmune disease.

### FACS analysis

PBMCs were isolated from freshly drawn blood samples by layering 20-25ml EDTA blood on 15ml Lymphoprep^{™} density gradient medium (Serumwerk Bernburg AG) and centrifugation at 22°C with 530g (1500 rpm) for 30 minutes. Washing was performed in three steps with the cell layer, with cold (4°C) DPBS/ EDTA (dulbecco's phosphate buffered saline), starting with careful centrifugation without brake at 4°C 300g for 15 minutes and two further centrifugations at 4°C 200g with brake for 10 minutes, with 35ml and 50ml DPBS/ EDTA. Isolated PBMCs were frozen in 15.5% albumin, 5% glucose and 10% dimethyl sulfoxide (DMSO). PBMC samples were stored at -80°C or in liquid nitrogen, whereas SNs and control medium were stored at -80°C. After thawing, PBMCs were incubated for one hour in RPMI 1640 medium, supplemented with 10% FCS, 1% penicillin/streptomycin as well as 1% L-glutamine. Ca. 8 x 106 cells were stimulated for 24h with 1% of the elite SN-E1 an SN-H1 in comparison to 1% control medium (MCDA). Subsequently, cells were washed in PBS and human TruStain FcX (BioLegend) was applied. For antibody staining a 1:200 dilution of eBioscience^{™} Fixable Viability Dye eFluor^{™} 780 (Invitrogen) and the described antibodies were used (Suppl. Tab. 1b). Intracellular staining was performed after permeabilization and fixation of cells with eBioscience^{™} Foxp3/Transcription Factor Staining Buffer Set (Invitrogen). Therefore, antibodies were diluted 1:100. Measurements were conducted on a BD LSRFortessa Cell Analyzer (BD Biosciences) with subsequent analyses using FlowJo 10.8.1 (BioSciences).

### Gene expression analysis

PBMCs of three healthy donors and three melanoma patients, isolated as described before, were cultured with 1000µl PBMC medium (see above). After incubation, cells were stimulated with SN-E1 and SN-H1 and control medium (MCDA) for 24h (1%) (10 µl of SN-E1 and SN-H1 or control medium). After 24h of incubation (37°C/ 5% CO2), RNA was isolated using the QIAGEN RNeasy Mini Kit (QIAGEN) with added b-Mercaptoethanol (10µl). After digestion on the column via RNase-Free DNase Set (QIAGEN), RNA gene expression analysis was performed using the nCounter SPRINT Profiler (NanoString Technologies, Inc.). For each sample, 75ng of RNA were used for hybridization for 20h (BIORAD, C1000 Touch Thermal Cycler). The used nCounter PanCancer Immune Profiling Panel^{™} (human) (Nanostring, XT_PGX_HuV1_CancerImmune_CSO) included 770 genes.

Statistical analysis of gene expression results was implemented with the nSolver Analysis Software (NanoString Technologies, Inc.) and ROSALIND^{®} (ROSALIND, Inc., San Diego, CA) (https://rosalind.bio/). In ROSALIND, housekeeping probes for normalization were selected by geNorm algorithm as in the NormqPCR R library [27], p-values (significance level p≤0.05) were adjusted by Benjamini-Hochberg method for false discovery rate estimation, heatmaps were created using PAM (Partitioning Around Medoids) method and the fpc R library [28]. Pathway analysis was conducted using EnrichR and EnrichR-KG, applying the databases KEGG 2021 Human and GO Biological Process 2021 [29,30].

### Stool Sample Collection and Sequencing

Stool samples were collected from both Elite and HPP patients to analyze the microbiome's role in ICI therapy responses. DNA was extracted from the stool samples, and next-generation sequencing (NGS) was performed to sequence the 16S rRNA gene. The microbiome composition was analyzed for differences between the two patient groups, focusing on specific taxa that could correlate with the clinical outcomes of ICI therapy. Sequencing data were processed using bioinformatics tools to identify microbial signatures that might influence the immune system's response to therapy.

### Results of proof-of-concept study:

Mographics and Clinical Data of Patients:
To characterize effects of the gut microbiome on melanoma cells and immune cells, supernatants from stool cultures of patients with metastatic melanoma showing an exceptional survival were used. Of 5 elite patients, with survival of 42-90 months after receiving ICI-therapy for stage IV metastatic disease, xy developed a complete response (CR) and xy a partial response (PR) according to RECIST 1.1. (Table 1). Tolerability of ICI was good, with moderate irAEs in xy out of 12 elite patients. Patient A showed the most prolonged PFS since the first application of ICI with xy months (range 6-80).

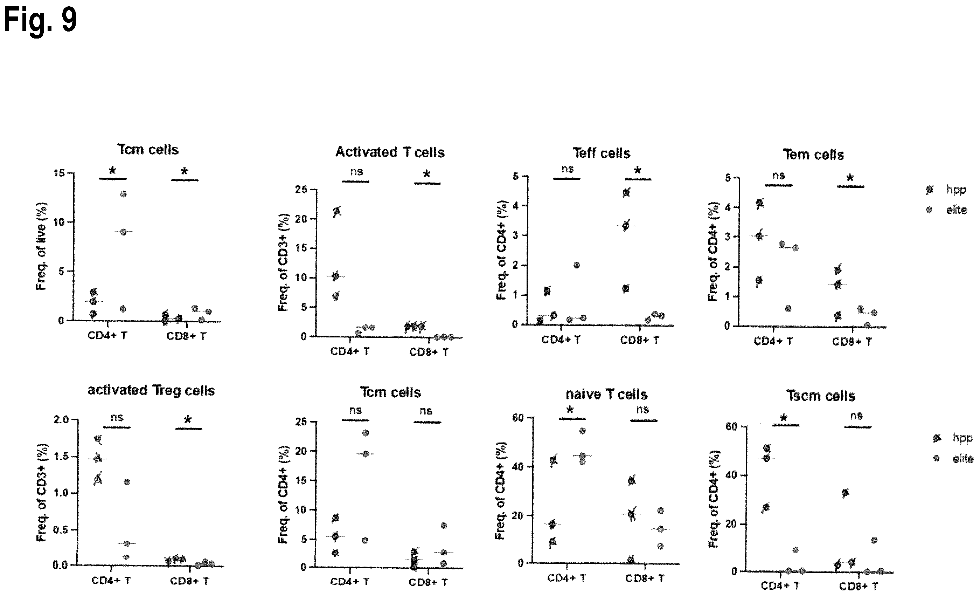

Immune Cell Activation and Exhaustion Markers Induced by Fecal Supernatants from Elite and HPP Melanoma Patients:
Flow cytometry analysis was performed to investigate the effects of fecal SN from melanoma patients classified as Elite (also referred to as "responders" or "R") and HPP (also referred to as "non-responders" or "NR") on immune cell activation. Fecal SN from HPP patients induced a stronger activation of CD8+ T cells, with significant increases in effector (Teff) and memory (Tem) T cells compared to Elite SN. This suggests that HPP SN provoke a more robust early immune response (Fig. 7a). However, HPP SN also upregulated markers of immune exhaustion, such as LAG3, TIGIT, and CTLA4, indicating a shift toward immune dysfunction despite the initial immune activation (Fig. 7b). Additionally, no significant differences were found in the activation of regulatory T cells (Tregs), naïve T cells, or stem T cells between the two groups. Both groups exhibited similar levels of activated Tregs, as well as comparable frequencies of naïve and stem T cells, suggesting that these T cell subsets did not contribute to the observed differences in immune response between HPP and Elite groups (Suppl. Fig. 9a).

In contrast, fecal supernatants from Elite patients resulted in a more balanced immune activation. CD8+ T cell activation was efficient, but there was no significant upregulation of exhaustion markers, including (TIGIT; CTLA-4, TIM-3, LAG-3) (Suppl. Fig. 9b), suggesting that the immune responses in the Elite group were more sustained and potentially more effective, without signs of immune exhaustion. Both HPP and Elite supernatants similarly enhanced the activation of antigen-presenting cells, specifically dendritic cells (DCs) and B cells, as evidenced by the upregulation of CD86 and HLA-DR (Fig. 7c). However, no significant differences were observed between the two groups in terms of DC activation markers (CD80, PD-L1) or B cell activation (Suppl. Fig. 9c). These findings indicate that while both HPP and Elite supernatants induce immune cell activation, the response in HPP patients was characterized by a higher likelihood of immune exhaustion, whereas Elite supernatants resulted in more sustained and balanced immune activation without signs of immune dysregulation.

Differential Immune Pathway Activation Induced by Fecal Supernatants from Elite and HPP Melanoma Patients: Nanostring Gene Expression Analysis:
A heatmap of the Nanostring data clearly demonstrated two distinct clusters separating the Elite and HPP groups, providing a visual representation of the differential gene expression profiles (Fig. 4). After 24-hour stimulation of PBMCs with 1% fecal SN from the Elite and HPP groups, Nanostring gene expression analysis revealed significant differences in immune-related gene expression between the two groups. A total of 81 genes were upregulated and 39 genes downregulated in PBMCs stimulated with Elite-SN compared to HPP-SN. Upregulated genes in the Elite group included AIRE (autoimmune regulator), FCGR2A (transporter functions), CCL25 (complement), TAP1 (antigen processing), and IFNAR1 (pathogen response), highlighting immune modulation and pathogen recognition pathways (Fig. 3). In terms of signaling pathways, the Elite group exhibited significant enrichment in cytokine-mediated signaling pathways (GO:0019221, p = 9.26E-25), natural killer (NK) cell-mediated cytotoxicity (p = 2.32E-17), and regulation of immune responses (GO:0050776, p = 1.59E-16), which are essential for immune activation and response to infection. Additional enriched pathways included Th17 cell differentiation (p = 1.52E-10) and cellular response to cytokine stimulus (GO:0071345, p = 2.14E-10), which further highlight immune cell activation and cytokine signaling.

In contrast, the HPP group also exhibited upregulation in cytokine-mediated signaling pathways (p = 1.44E-20) and TNF signaling (p = 1.12E-10), suggesting immune activation. However, there were key differences in immune modulation between the two groups. The downregulated gene pathways in the Elite group compared to the HPP group included immune signaling through BCL10 (immune signaling adaptor), CASP3 (cell apoptosis), JAK2 (cytokine and growth factor signaling), IL15 and TNFSF14 (regulation), and EGR1 (T cell function) (Fig. 3). These downregulated genes point to a more controlled immune response in the Elite group, with reduced inflammatory signaling and apoptosis. Overall, these results indicate that Elite-SN promotes a more coordinated and sustained immune response, characterized by a strong upregulation of immune activation and cytokine production, while HPP-SN supports a less effective, potentially dysregulated immune environment. This data underscores the complex role of the gut in modulating immune responses and their potential influence on cancer immunotherapy outcomes.

### Discussion:

Whereas recent research mainly focused on the composition of the beneficial or detrimental microbiome with respect to cancer and immunotherapy [1,3], there is a dire need to unravel the mechanisms of action of the microbiome on immunotherapy. This study investigated a new method to unravel effects of the gut microbiome of long-term melanoma survivors undergoing immunotherapy on tumor and immune cells. This model was established using supernatants of stool cultures from such elite patients, and demonstrated anti-proliferative effects on tumor cells and activation of the immune system in vitro as assessed by immunophenotyping and gene expression analyses of PBMCs from the elected melanoma patients and healthy controls.

This study aimed to investigate and establish a new model as an alternative to previous microbiome modulating approaches.

SN-stimulation induced elevated expression of p-AKT and p-ERK in BRAF-V600E mutated melanoma cells. Interestingly, IFNy-dependent activation of ERK is relevant for cell death induction in melanoma [32]. Our results indicate activation of ERK in the context of SN-stimulation to represent an alternative way to induce ERK-dependent melanoma cell death.

SN impacted genes related to cytokine regulation, -interaction and inflammatory response as shown by gene expression and pathway analyses, as well as genes related to activation and proliferation of T- and B-cells and macrophages.

Using flow cytometry, we demonstrated an association of T-cell activation with SN-stimulation, with elevated frequencies of CD3+, CD4+ and CD8+ T-cells. Furthermore, SN induced an increased expression of CD25, CD44 and ICOS on CD3+, CD4+ and CD8+ T-cells, and increased frequencies of CTLA-4+ CD8+ T-cells, which have been shown to contribute to improved response to ICI-therapy [39,40]. SN increased the activation of B-cells, with elevated expression of CD86 as activation marker, which has been associated with improved survival and high abundance of CD8+ T-cells in tertiary lymphoid structures [41,42]. Additionally, SN-stimulation led to increased expression of PD-L1 on macrophages and higher frequency of CD14+ HLA-DR+ monocytes, which have been associated with prolonged PFS and overall survival (OS) [43,44]. Dendritic cells became activated by SN, with increased frequencies of CD80 and HLA-DR expressing DCs.

### Tables:

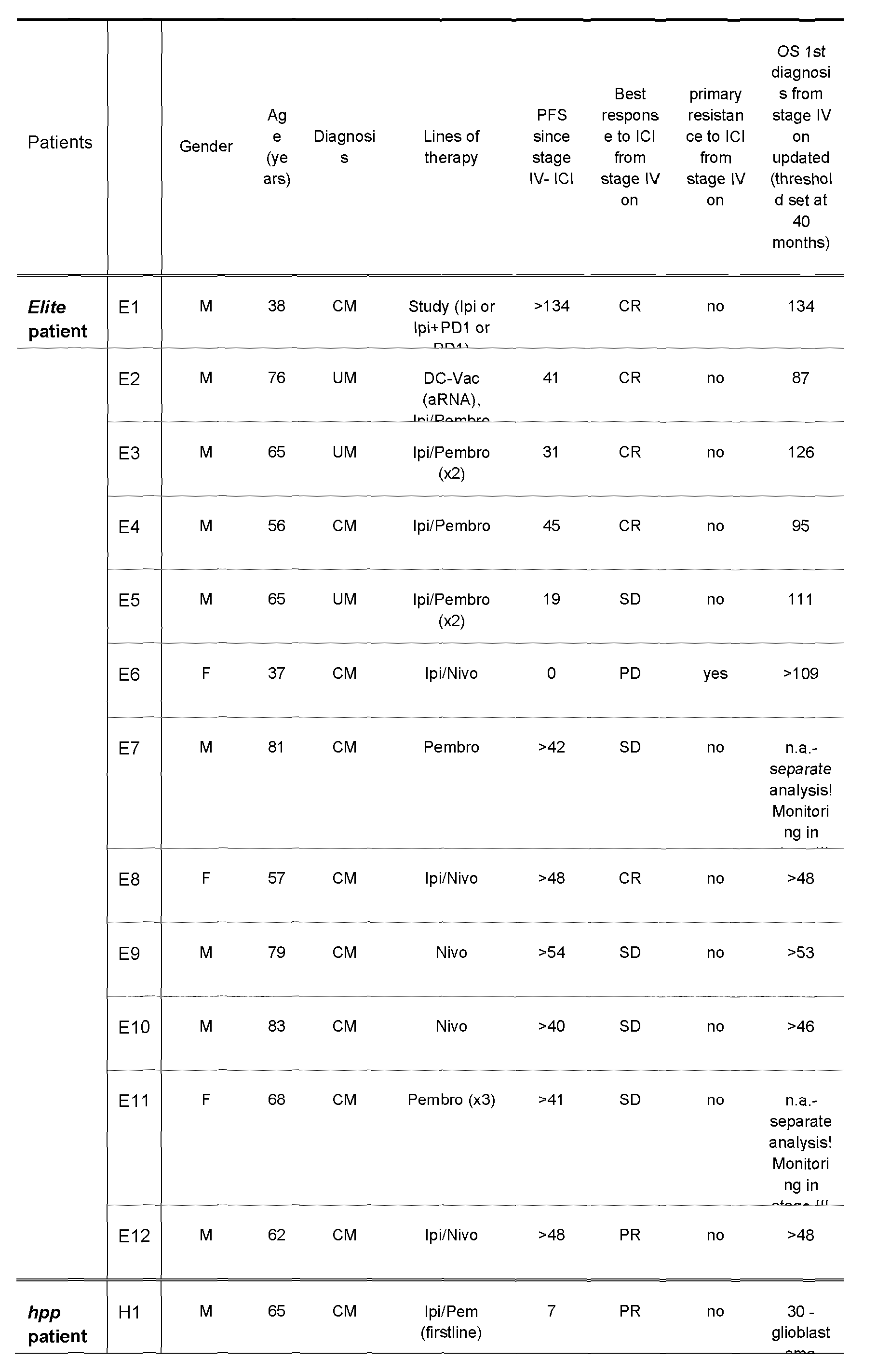

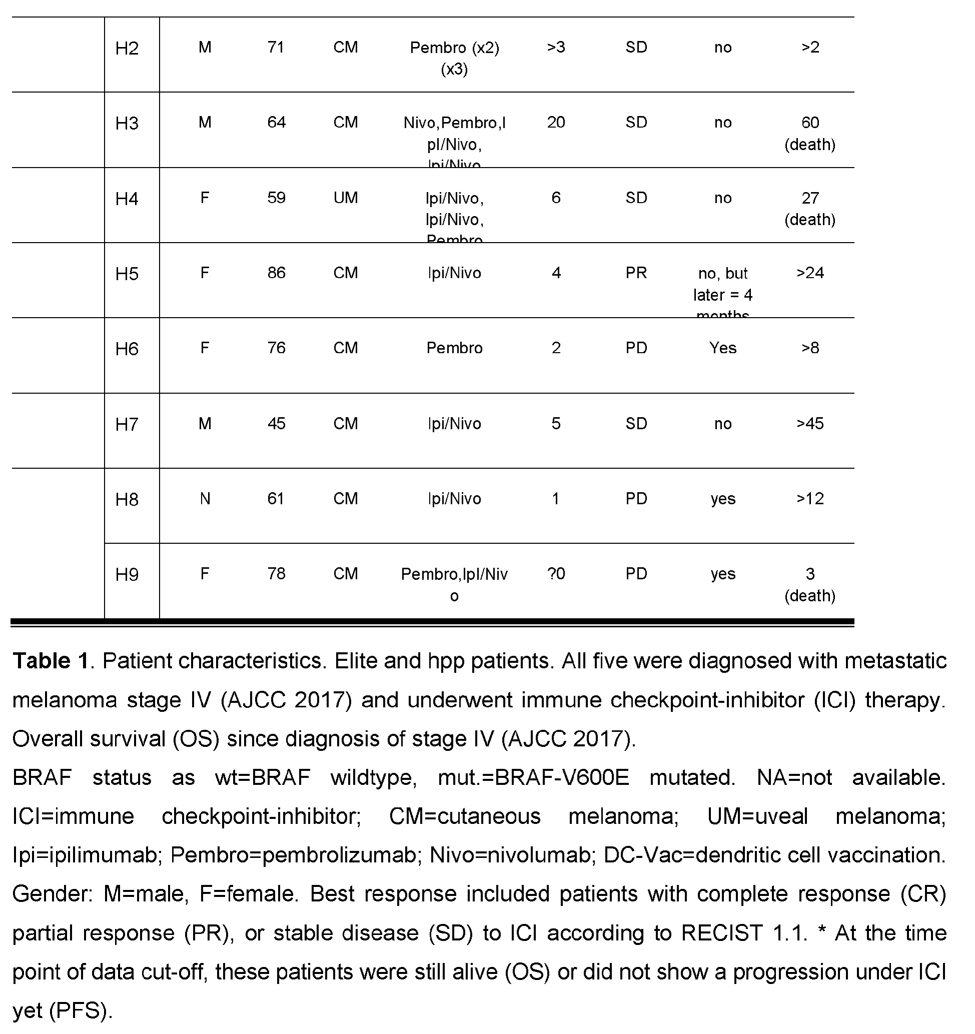

### Overall survival (OS) since diagnosis of stage IV (AJCC 2017).

BRAF status as wt=BRAF wildtype, mut.=BRAF-V600E mutated. NA=not available. ICI=immune checkpoint-inhibitor; CM=cutaneous melanoma; UM=uveal melanoma; lpi=ipilimumab; Pembro=pembrolizumab; Nivo=nivolumab; DC-Vac=dendritic cell vaccination. Gender: M=male, F=female. Best response included patients with complete response (CR) partial response (PR), or stable disease (SD) to ICI according to RECIST 1.1. * At the time point of data cut-off, these patients were still alive (OS) or did not show a progression under ICI yet (PFS).

**Table 2. Melanoma patients - PBMC donors. OS since first initiation of ICI therapy displayed. Stages at ICI initiation were IIIB for patients 1 and 2 and stage IV (AJCC 2017) for patient 3, who was the only PBMC donor with prior systemic therapy before ICI initiation, with BRAF/MEK inhibitors. OS and PFS in months. Numbers of re-initiations of a line of therapy displayed in brackets (x2, x3) if re-initiations followed consecutively.**

| **Melanoma patients -** | **1** | **F** | **72** | **CM** | **2** | **Pembro** | **wt** | **PR** | **16** | **23*** |
|---|---|---|---|---|---|---|---|---|---|---|
| | 2 | F | 65 | CM | 0 | Pembro (x3) | mut. | SD | 17* | 17* |
| | 3 | M | 69 | CM | 3 | Ipi/Nivo (x2) | mut. | SD | 11* | 11* |

BRAF status as wt=BRAF wildtype, mut.=BRAF-V600E mutated. NA=not available. ICI=immune checkpoint-inhibitor; CM=cutaneous melanoma; UM=uveal melanoma; lpi=ipilimumab; Pembro=pembrolizumab; Nivo=nivolumab; DC-Vac=dendritic cell vaccination. Gender: M=male, F=female. Best response included patients with complete response (CR) partial response (PR), or stable disease (SD) to ICI according to RECIST 1.1. * At the time point of data cut-off, these patients were still alive (OS) or did not show a progression under ICI yet (PFS).

### List of abbreviations

- BCA: Bicinchoninic acid
- Co, Ctrl: Control
- CR: Complete response
- CTLA-4: Cytotoxic T-lymphocyte-associated Protein 4
- DMSO: Dimethyl sulfoxide
- ECL: Enhanced chemiluminescence
- FACS: Fluorescence Activated Cell Sorting
- FMT: Fecal microbiota transplantation
- ICI: Immune-checkpoint inhibitor
- irAE: Immune-related adverse event
- MC: Melanoma cell cultures
- MCDA: Control medium, used for supernatant culturing
- Mel: Melanoma patient
- MFI: Mean fluorescence intensity
- OS: Overall survival
- PBMCs: Peripheral blood mononuclear cells
- PD: Progressive disease
- PD-1: Programmed death-receptor 1
- PFS: Progression free survival
- PR: Partial response
- SD: Stable disease
- SN: Supernatant
- TMAO: Trimethylamine N-oxide

### References

1. McCulloch, J.A., et al. Intestinal microbiota signatures of clinical response and immune-related adverse events in melanoma patients treated with anti-PD-1. Nature medicine 28, 545-556 (2022).
2. Routy, B., et al. Gut microbiome influences efficacy of PD-1-based immunotherapy against epithelial tumors. Science (New York, N.Y.) 359, 91-97 (2018).
3. Gopalakrishnan, V., et al. Gut microbiome modulates response to anti-PD-1 immunotherapy in melanoma patients. Science (New York, N.Y.) 359, 97-103 (2018).
4. Matson, V., et al. The commensal microbiome is associated with anti-PD-1 efficacy in metastatic melanoma patients. Science (New York, N.Y.) 359, 104-108 (2018).
5. Chaput, N., et al. Baseline gut microbiota predicts clinical response and colitis in metastatic melanoma patients treated with ipilimumab. Annals of oncology : official journal of the European Society for Medical Oncology 28, 1368-1379 (2017).
6. Derosa, L., et al. Intestinal Akkermansia muciniphila predicts clinical response to PD-1 blockade in patients with advanced non-small-cell lung cancer. Nature medicine 28, 315-324 (2022).
7. Meza, L.A., et al. A phase I trial to evaluate the biologic effect of CBM588 (Clostridium butyricum) in combination with cabozantinib plus nivolumab for patients with metastatic renal cell carcinoma (mRCC). 40, TPS4606-TPS4606 (2022).
8. Lee, K.A., et al. Cross-cohort gut microbiome associations with immune checkpoint inhibitor response in advanced melanoma. Nature medicine 28, 535-544 (2022).
9. Baruch, E.N., et al. Fecal microbiota transplant promotes response in immunotherapy-refractory melanoma patients. Science (New York, N.Y.) 371, 602-609 (2021).
10. Davar, D., et al. Fecal microbiota transplant overcomes resistance to anti-PD-1 therapy in melanoma patients. Science (New York, N.Y.) 371, 595-602 (2021).
11. Routy, B., et al. Fecal microbiota transplantation plus anti-PD-1 immunotherapy in advanced melanoma: a phase I trial. Nature medicine 29, 2121-2132 (2023).
12. Kim, G., et al. Fecal microbiota transplantation combined with anti-PD-1 inhibitor for unresectable or metastatic solid cancers refractory to anti-PD-1 inhibitor. 41, 105-105 (2023).
13. Cammarota, G., et al. European consensus conference on faecal microbiota transplantation in clinical practice. Gut 66, 569-580 (2017).
14. Huang, J.T. & Mao, Y.Q. The impact of the microbiome in cancer: Targeting metabolism of cancer cells and host. Frontiers in oncology 12, 1029033 (2022).
15. Dekkers, K.F., et al. An online atlas of human plasma metabolite signatures of gut microbiome composition. Nature communications 13, 5370 (2022).
16. Lee-Sarwar, K.A., Lasky-Su, J., Kelly, R.S., Litonjua, A.A. & Weiss, S.T. Metabolome-Microbiome Crosstalk and Human Disease. Metabolites 10(2020).
17. Peyraud, F., et al. Circulating L-arginine predicts the survival of cancer patients treated with immune checkpoint inhibitors. Annals of oncology : official journal of the European Society for Medical Oncology 33, 1041-1051 (2022).
18. Nomura, M., et al. Association of Short-Chain Fatty Acids in the Gut Microbiome With Clinical Response to Treatment With Nivolumab or Pembrolizumab in Patients With Solid Cancer Tumors. JAMA network open 3, e202895 (2020).
19. Mager, L.F., et al. Microbiome-derived inosine modulates response to checkpoint inhibitor immunotherapy. Science (New York, N.Y.) 369, 1481-1489 (2020).
20. Lee, P.C., et al. Gut microbiota and metabolites associate with outcomes of immune checkpoint inhibitor-treated unresectable hepatocellular carcinoma. Journal for immunotherapy of cancer 10(2022).
21. Alexander, E.T., et al. Harnessing the polyamine transport system to treat BRAF inhibitor-resistant melanoma. Cancer biology & therapy 22, 225-237 (2021).
22. Valko-Rokytovská, M., et al. Specific Urinary Metabolites in Malignant Melanoma. Medicina (Kaunas, Lithuania) 55(2019).
23. Frankel, A.E., et al. Metagenomic Shotgun Sequencing and Unbiased Metabolomic Profiling Identify Specific Human Gut Microbiota and Metabolites Associated with Immune Checkpoint Therapy Efficacy in Melanoma Patients. Neoplasia 19, 848-855 (2017).
24. Fischer, G.M., et al. Molecular Profiling Reveals Unique Immune and Metabolic Features of Melanoma Brain Metastases. Cancer discovery 9, 628-645 (2019).
25. Triozzi, P.L., et al. Circulating Immune Bioenergetic, Metabolic, and Genetic Signatures Predict Melanoma Patients' Response to Anti-PD-1 Immune Checkpoint Blockade. Clinical cancer research : an official journal of the American Association for Cancer Research 28, 1192-1202 (2022).
26. Domingo, M.C., Huletsky, A., Giroux, R., Picard, F.J. & Bergeron, M.G. vanD and vanG-like gene clusters in a Ruminococcus species isolated from human bowel flora. Antimicrobial agents and chemotherapy 51, 4111-4117 (2007).
27. Perkins, J.R., et al. ReadqPCR and NormqPCR: R packages for the reading, quality checking and normalisation of RT-qPCR quantification cycle (Cq) data. BMC genomics 13, 296 (2012).
28. Hennig, C. Cran-package fpc..
29. Xie, Z., et al. Gene Set Knowledge Discovery with Enrichr. Current protocols 1, e90 (2021).
30. Evangelista, J.E., et al. Enrichr-KG: bridging enrichment analysis across multiple libraries. Nucleic Acids Research 51, W168-W179 (2023).
31. Carpenter, E.L., et al. Mitochondrial complex I inhibitor deguelin induces metabolic reprogramming and sensitizes vemurafenib-resistant BRAF(V600E) mutation bearing metastatic melanoma cells. Molecular carcinogenesis 58, 1680-1690 (2019).
32. Champhekar, A., et al. ERK mediates interferon gamma-induced melanoma cell death. Molecular cancer 22, 165 (2023).
33. Manguso, R.T., et al. In vivo CRISPR screening identifies Ptpn2 as a cancer immunotherapy target. Nature 547, 413-418 (2017).
34. Davies, M.A., et al. Integrated Molecular and Clinical Analysis of AKT Activation in Metastatic Melanoma. Clinical cancer research : an official journal of the American Association for Cancer Research 15, 7538-7546 (2009).
35. Vergadi, E., leronymaki, E., Lyroni, K., Vaporidi, K. & Tsatsanis, C. Akt Signaling Pathway in Macrophage Activation and M1/M2 Polarization. Journal of immunology (Baltimore, Md. : 1950) 198, 1006-1014 (2017).
36. Khunger, A., et al. CTLA-4 blockade and interferon-α induce proinflammatory transcriptional changes in the tumor immune landscape that correlate with pathologic response in melanoma. PloS one 16, e0245287 (2021).
37. Krieg, C., et al. Complement downregulation promotes an inflammatory signature that renders colorectal cancer susceptible to immunotherapy. Journal for immunotherapy of cancer 10(2022).
38. Yan, K., Lu, Y., Yan, Z. & Wang, Y. 9-Gene Signature Correlated With CD8(+) T Cell Infiltration Activated by IFN-γ: A Biomarker of Immune Checkpoint Therapy Response in Melanoma. Frontiers in immunology 12, 622563 (2021).
39. Mastracci, L., et al. Response to ipilimumab therapy in metastatic melanoma patients: potential relevance of CTLA-4(+) tumor infiltrating lymphocytes and their in situ localization. Cancer immunology, immunotherapy: CII 69, 653-662 (2020).
40. Chen, B.-J., Zhao, J.-W., Zhang, D.-H., Zheng, A.-H. & Wu, G.-Q. Immunotherapy of Cancer by Targeting Regulatory T cells. International Immunopharmacology 104, 108469 (2022).
41. Helmink, B.A., et al. B cells and tertiary lymphoid structures promote immunotherapy response. Nature 577, 549-555 (2020).
42. Wennhold, K., et al. CD86(+) Antigen-Presenting B Cells Are Increased in Cancer, Localize in Tertiary Lymphoid Structures, and Induce Specific T-cell Responses. Cancer immunology research 9, 1098-1108 (2021).
43. Toki, M.I., et al. High-Plex Predictive Marker Discovery for Melanoma Immunotherapy-Treated Patients Using Digital Spatial Profiling. Clinical cancer research : an official journal of the American Association for Cancer Research 25, 5503-5512 (2019).
44. Krieg, C., et al. High-dimensional single-cell analysis predicts response to anti-PD-1 immunotherapy. Nature medicine 24, 144-153 (2018).
45. Sosa Cuevas, E., Saas, P. & Aspord, C. Dendritic Cell Subsets in Melanoma: Pathophysiology, Clinical Prognosis and Therapeutic Exploitation. Cancers 15(2023).
46. Lam, K.C., et al. Microbiota triggers STING-type I IFN-dependent monocyte reprogramming of the tumor microenvironment. Cell 184, 5338-5356.e5321 (2021).
47. Feng, Q., et al. Lactate increases stemness of CD8 + T cells to augment anti-tumor immunity. Nature communications 13, 4981 (2022).
48. Zhang, L., et al. Inhibition of UBA6 by inosine augments tumour immunogenicity and responses. Nature communications 13, 5413 (2022).
49. Mirji, G., et al. The microbiome-derived metabolite TMAO drives immune activation and boosts responses to immune checkpoint blockade in pancreatic cancer. Science immunology 7, eabn0704 (2022).

Aspects of the present invention are described in the following clauses:
Clauses
1. An *in vitro* method for providing information for predicting a subject's responsiveness to and/or benefit from immunotherapy and/or the subject's likelihood of progression, occurrence or recurrence of disease, comprising:
   a) generating a stool-based sample of the subject;
   b) stimulating cells comprising or consisting of peripheral blood mononuclear cells (PBMCs) with the stool-based sample;
   c) performing a gene expression analysis of the stimulated PBMCs;
   d) using the results of the gene expression analysis as said information.
2. Method according to clause 1 wherein the stool-based sample is a stool sample or a cultured stool sample, or a fraction thereof, in particular a supernatant fraction thereof.
3. Method according to clause 1 or 2, wherein whole blood or a fraction thereof is used as the cells comprising or consisting of peripheral blood mononuclear cells (PBMCs).
4. Method according to any of the preceding clauses, wherein a stool sample is obtained from the subject, dispersed in a liquid medium, optionally cultured, and a supernatant thereof is obtained and used in step b) of clause 1, and wherein optionally the supernatant is cell-free and/or sterile-filtered.
5. Method according to any of the preceding clauses, wherein the stool-based sample, in particular the supernatant according to clause 4, is used in the stimulating according to step b) at a concentration of 0.1 to 20 vol.-%, in particular 1 to 10 vol.-% based on the total volume of the sample containing the cells comprising or consisting of peripheral blood mononuclear cells (PBMCs).
6. Method according to any of the preceding clauses, wherein the stimulating according to step b) of clause 1 is performed from about 0.1 to about 48 h, in particular from about 1 to 24 h.
7. Method according to any of the preceding clauses, wherein the immunotherapy is a cancer immunotherapy, in particular a checkpoint inhibitor therapy.
8. Method according to any of the preceding clauses, wherein the disease is a cancer, an inflammatory disease such as inflammatory bowel disease, a metabolic syndrome, or an autoimmune disorder.
9. Method according to any of the preceding clauses, wherein the cancer is one or more selected from the group consisting of melanoma, lung cancer, cholangiocarcinoma, colon cancer, adenocarcinoma, rectal cancer, breast cancer, synovial sarcoma, chondrosarcoma, thyroid cancer, stomach cancer, thymus cancer, cervical cancer, glioma, brain cancer, bladder cancer, prostate cancer, leukemia, kidney cancer, liver cancer, colorectal cancer, pancreatic cancer, ovarian cancer, lymphoma, uterine cancer, oral cancer, bronchial cancer, nasopharyngeal cancer, laryngeal cancer, skin cancers, blood cancer, parathyroid cancer, and ureteral cancer.
10. Method according to any of the preceding clauses, wherein the gene expression analysis performed is a gene expression quantification using quantitative real-time PCR (qRT-PCR) or RNA sequencing.
11. Method according to any of the preceding clauses, wherein gene expression analysis performed comprises analysis of genes involved in tumor proliferation, immune response, angiogenesis, and apoptosis.
12. Method according to any of the preceding clauses, wherein the information used for predicting responsiveness includes
   the gene expression analysis results for at least one of the group of AIRE (autoimmune regulator), FCGR2A (transporter functions), CCL25 (complement), TAP1 (antigen processing), IFNAR1 (pathogen response), BST2 (protein coding) and CSF2RB (cytokine receptor), and/or
   the gene expression analysis results for at least one of the group consisting of BCL10 (immune signalling adaptor), CASP3 (cell apoptosis), JAK2 (cytokine and growth factor signalling), IL15 and TNFSF14 (regulation), EGR1 (early growth response), IGF2R (tumor growth, apoptosis, angiogenesis, and invasion) and UBC (cell survival, proliferation, sensitivity to treatment).
13. Method according to any of the preceding clauses, wherein the method includes
   - first performing the gene expression analysis as described in steps a) to c) of clause 1 on one or more patients who are non-responders NR to immune therapy in order to obtain one or more NR gene expression signatures;
   - comparing the NR gene expression signature(s) to the results of the gene expression analysis of the subject's stool-based sample stimulated PBMCs according to clause 1;
   - determining the expected responsiveness of the subject to immunotherapy and/or the likelihood of developing or progressing with a disease, based on a result of said comparison.
14. Method according to any of the preceding clauses, wherein the method includes
   - first performing the gene expression analysis as described in steps a) to c) of clause 1 on one or more patients who are high responders R to immune therapy in order to obtain one or more R gene expression signatures;
   - comparing the R gene expression signature(s) to the results of the gene expression analysis of the subject's stool-based sample stimulated PBMCs according to clause 1;
   - determining the expected responsiveness of the subject to immunotherapy and/or the likelihood of developing or progressing with a disease, based on a result of said comparison.
15. Method according to clause 13 or clause 14, wherein the comparison comprises gene expression analysis results, especially respectively, for at least one of the group of AIRE (autoimmune regulator), FCGR2A (transporter functions), CCL25 (complement), TAP1 (antigen processing), IFNAR1 (pathogen response), BST2 and CSF2RB,
   and/or for at least one of the group consisting of BCL10 (immune signalling adaptor), CASP3 (cell apoptosis), JAK2 (cytokine and growth factor signalling), IL15 and TNFSF14 (regulation), EGR1, IGF2R and UBC.
16. Method according to clause 15 with clause 13, wherein the subject is grouped as a predicted responder to immunotherapy and/or having a low risk of occurrence, progression or recurrence of disease if, compared to the NR gene expression signature,
   - the subject's stool-based sample stimulated PBMCs have an upregulated gene expression of at least one of the group consisting of AIRE (autoimmune regulator), FCGR2A (transporter functions), CCL25 (complement), TAP1 (antigen processing), IFNAR1 (pathogen response), BST2 and CSF2RB, and/or
   - the subject's stool-based sample stimulated PBMCs have a downregulated gene expression of at least one of the group consisting of and/or for at least one of the group consisting of BCL10 (immune signalling adaptor), CASP3 (cell apoptosis), JAK2 (cytokine and growth factor signalling), IL15 and TNFSF14 (regulation), EGR1, IGF2R and UBC.
17. Method according to clause 15 with clause 14, wherein the subject is grouped as a predicted non-responder to immunotherapy and/or having a high risk of occurrence, progression or recurrence of a disease if, compared to the R gene expression signature,
   - the subject's stool-based sample stimulated PBMCs have a downregulated gene expression of at least one of the group consisting of AIRE (autoimmune regulator), FCGR2A (transporter functions), CCL25 (complement), TAP1 (antigen processing), IFNAR1 (pathogen response), BST2 and CSF2RB, and/or
   - the subject's stool-based sample stimulated PBMCs have an upregulated gene expression of at least one of the group consisting of and/or for at least one of the group consisting of BCL10 (immune signalling adaptor), CASP3 (cell apoptosis), JAK2 (cytokine and growth factor signalling), IL15 and TNFSF14 (regulation), EGR1, IGF2R and UBC.
18. A method for classifying the likelihood of responsiveness of a subject to immunotherapy and/or the likelihood of occurrence or progression or recurrence of a disease, comprising
   e) performing the method according to any one of clauses 1 to 17 in order to obtain information for predicting responsiveness and/or the likelihood of developing or progressing with a disease,
   f) using the information for predicting responsiveness and/or likelihood in order to obtain a response score,
      wherein a higher response score is associated with an increased responsiveness to immunotherapy and/or decreased likelihood of developing or progressing with a disease, and a lower response score is associated with a decreased responsiveness to immunotherapy and/or increased likelihood of developing or progressing with a disease.
19. A method for selecting the mode of treatment for a subject or patient, the method comprising using the response score generated in Clause 18 generated as follows:
   - providing a specific immunotherapy to the subject or patient if the subject or patent has a "high" response score;
   - providing an alternative therapy, another immunotherapy or a therapy in addition to a specific immunotherapy to a subject or patient having a "low" response score.
20. A kit for *in vitro* determination of a gene expression profile of cells comprising or consisting of peripheral blood mononuclear cells (PBMCs) stimulated with a stool-based sample, the kit comprising:
   - reagents for obtaining a stool-based sample from a subject;
   - reagents for culturing cells comprising or consisting of peripheral blood mononuclear cells (PBMCs) stimulated with the stool-based sample;
   - reagents for determining gene expression profiles of the PBMCs stimulated with the stool-based sample;
   - instructions for generating a response score for immunotherapy from the gene expression profiles of the PBMCs stimulated with the stool-based sample.
21. Use of a method according to clause 1 and/or a kit according to Clause 20 for predicting the response of a subject or patient to immunotherapy, classifying the likelihood of a patient responding to immunotherapy, and/or selecting the mode of treatment for the subject or patient.
22. Use of a stool-based sample from a subject for stimulating cells comprising or consisting of peripheral blood mononuclear cells (PBMCs) and obtaining a gene expression analysis of the stimulated PBMCs.
23. Use according to the preceding clause wherein the results of the gene expression analysis are used as information within the prediction of and/or classification of the responsiveness of the subject for immunotherapy and/or the likelihood of developing or progressing with a disease, and/or for selecting the mode of treatment of the subject.

By the foregoing described embodiments and clauses, an in vitro method for providing information for predicting a subject's responsiveness to and benefit from immunotherapy and/or the subject's likelihood of progression or recurrence of disease is achieved.

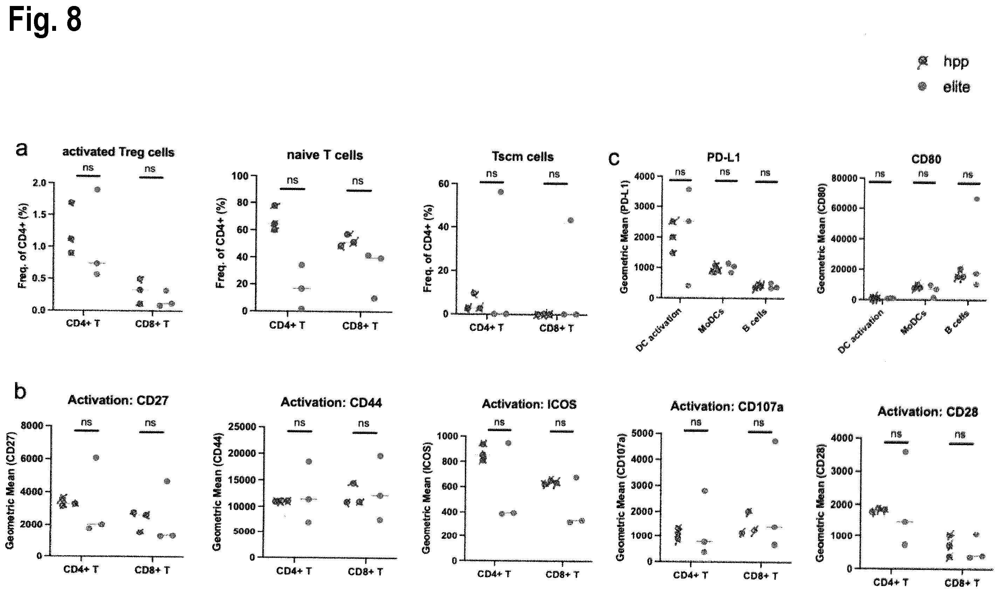

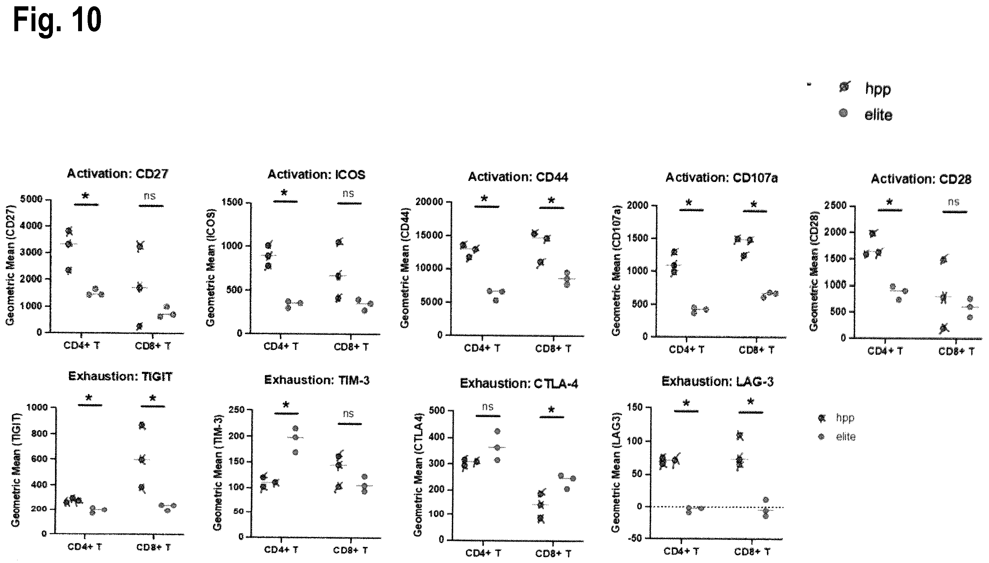

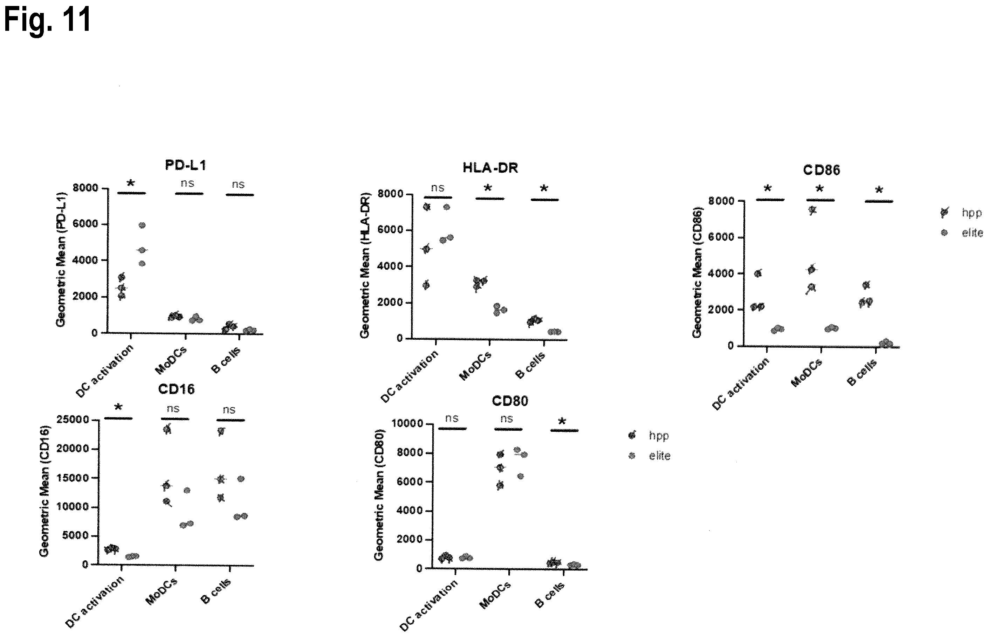

In addition to the foregoing written explanations, it is explicitly referred to figures 1 to 11 wherein the figures in detail show examples, methods, uses, and proof-of-concept results of the invention.

### List of Reference Numerals

- S0: selective preliminary step
- S1: obtaining stool-sample
- S2: generating stool-based sample of the subject
- S3: stimulating PBMCs with stool-based sample
- S4: performing gene expression analysis of the stimulated PBMCs
- S5: using the results of the gene expression analysis as information for predicting responsiveness of the subject to immunotherapy or the likelihood of developing/progression with an immune-related disease
- T1: threshold for up-regulation
- T2: threshold for down-regulation
- T3: threshold for adjust p-value

## Claims

1. An *in vitro* method for providing information for predicting a subject's responsiveness to and/or benefit from immunotherapy and/or the subject's likelihood of progression, occurrence or recurrence of disease, comprising:
a) generating a stool-based sample of the subject;
b) stimulating cells comprising or consisting of peripheral blood mononuclear cells (PBMCs) with the stool-based sample;
c) performing a gene expression analysis of the stimulated PBMCs;
d) using the results of the gene expression analysis as said information.

2. Method according to claim 1 wherein the stool-based sample is a stool sample or a cultured stool sample, or a fraction thereof, in particular a supernatant fraction thereof.

3. Method according to claim 1 or 2, wherein whole blood or a fraction thereof is used as the cells comprising or consisting of peripheral blood mononuclear cells (PBMCs).

4. Method according to any of the preceding claims, wherein a stool sample is obtained from the subject, dispersed in a liquid medium, optionally cultured, and a supernatant thereof is obtained and used in step b) of claim 1, and wherein optionally the supernatant is cell-free and/or sterile-filtered.

5. Method according to any of the preceding claims, wherein the stool-based sample, in particular the supernatant according to claim 4, is used in the stimulating according to step b) at a concentration of 0.1 to 20 vol.-%, in particular 1 to 10 vol.-% based on the total volume of the sample containing the cells comprising or consisting of peripheral blood mononuclear cells (PBMCs).

6. Method according to any of the preceding claims, wherein the immunotherapy is a cancer immunotherapy, in particular a checkpoint inhibitor therapy
, wherein the disease is especially a cancer, an inflammatory disease such as inflammatory bowel disease, a metabolic syndrome, or an autoimmune disorder.

7. Method according to any of the preceding claims, wherein the information used for predicting responsiveness includes
the gene expression analysis results for at least one of the group of AIRE (autoimmune regulator), FCGR2A (transporter functions), CCL25 (complement), TAP1 (antigen processing), IFNAR1 (pathogen response), BST2 (protein coding) and CSF2RB (cytokine receptor), and/or
the gene expression analysis results for at least one of the group consisting of BCL10 (immune signalling adaptor), CASP3 (cell apoptosis), JAK2 (cytokine and growth factor signalling), IL15 and TNFSF14 (regulation), EGR1 (early growth response), IGF2R (tumor growth, apoptosis, angiogenesis, and invasion) and UBC (cell survival, proliferation, sensitivity to treatment).

8. Method according to any of the preceding claims, wherein the method includes
- first performing the gene expression analysis as described in steps a) to c) of claim 1 on one or more patients who are non-responders NR to immune therapy in order to obtain one or more NR gene expression signatures;
- comparing the NR gene expression signature(s) to the results of the gene expression analysis of the subject's stool-based sample stimulated PBMCs according to claim 1;
- determining the expected responsiveness of the subject to immunotherapy and/or the likelihood of developing or progressing with a disease, based on a result of said comparison.

9. Method according to any of the preceding claims, wherein the method includes
- first performing the gene expression analysis as described in steps a) to c) of claim 1 on one or more patients who are high responders R to immune therapy in order to obtain one or more R gene expression signatures;
- comparing the R gene expression signature(s) to the results of the gene expression analysis of the subject's stool-based sample stimulated PBMCs according to claim 1;
- determining the expected responsiveness of the subject to immunotherapy and/or the likelihood of developing or progressing with a disease, based on a result of said comparison.

10. Method according to claim 8 or claim 9, wherein the comparison comprises gene expression analysis results, especially respectively, for at least one of the group of AIRE (autoimmune regulator), FCGR2A (transporter functions), CCL25 (complement), TAP1 (antigen processing), IFNAR1 (pathogen response), BST2 and CSF2RB,
and/or for at least one of the group consisting of BCL10 (immune signalling adaptor), CASP3 (cell apoptosis), JAK2 (cytokine and growth factor signalling), IL15 and TNFSF14 (regulation), EGR1, IGF2R and UBC.

11. Method according to claim 10 with claim 8, wherein the subject is grouped as a predicted responder to immunotherapy and/or having a low risk of occurrence, progression or recurrence of disease if, compared to the NR gene expression signature,
- the subject's stool-based sample stimulated PBMCs have an upregulated gene expression of at least one of the group consisting of AIRE (autoimmune regulator), FCGR2A (transporter functions), CCL25 (complement), TAP1 (antigen processing), IFNAR1 (pathogen response), BST2 and CSF2RB, and/or
- the subject's stool-based sample stimulated PBMCs have a downregulated gene expression of at least one of the group consisting of and/or for at least one of the group consisting of BCL10 (immune signalling adaptor), CASP3 (cell apoptosis), JAK2 (cytokine and growth factor signalling), IL15 and TNFSF14 (regulation), EGR1, IGF2R and UBC.

12. Method according to claim 10 with claim 9, wherein the subject is grouped as a predicted non-responder to immunotherapy and/or having a high risk of occurrence, progression or recurrence of a disease if, compared to the R gene expression signature,
- the subject's stool-based sample stimulated PBMCs have a downregulated gene expression of at least one of the group consisting of AIRE (autoimmune regulator), FCGR2A (transporter functions), CCL25 (complement), TAP1 (antigen processing), IFNAR1 (pathogen response), BST2 and CSF2RB, and/or
- the subject's stool-based sample stimulated PBMCs have an upregulated gene expression of at least one of the group consisting of and/or for at least one of the group consisting of BCL10 (immune signalling adaptor), CASP3 (cell apoptosis), JAK2 (cytokine and growth factor signalling), IL15 and TNFSF14 (regulation), EGR1, IGF2R and UBC.

13. A kit for *in vitro* determination of a gene expression profile of cells comprising or consisting of peripheral blood mononuclear cells (PBMCs) stimulated with a stool-based sample, the kit comprising:
- reagents for obtaining a stool-based sample from a subject;
- reagents for culturing cells comprising or consisting of peripheral blood mononuclear cells (PBMCs) stimulated with the stool-based sample;
- reagents for determining gene expression profiles of the PBMCs stimulated with the stool-based sample;
- instructions for generating a response score for immunotherapy from the gene expression profiles of the PBMCs stimulated with the stool-based sample.

14. Use of a stool-based sample from a subject for stimulating cells comprising or consisting of peripheral blood mononuclear cells (PBMCs) and obtaining a gene expression analysis of the stimulated PBMCs.

15. Use according to the preceding claim wherein the results of the gene expression analysis are used as information within the prediction of and/or classification of the responsiveness of the subject for immunotherapy and/or the likelihood of developing or progressing with a disease, and/or for selecting the mode of treatment of the subject.
